# EUROPEAN PATENT APPLICATION

(11) **EP 4 621 043 A1**
(43) Date of publication of application: **24.09.2025**
(21) Application number: 23891489.9
(22) Date of filing: 10.11.2023
(51) Int. Cl.: C12N 1/16, C12P 11/00, C12P 13/04

(54) **NOVEL MICROORGANISM, NOVEL MICROORGANISM CULTURE OR EXTRACT, AND ERGOTHIONEINE PRODUCTION METHOD**

(30) Priority: 18.11.2022 JP 2022185256
(71) Applicant: Kureha Corporation, Chuo-ku Tokyo 103-8552 (JP); National Institute of Advanced Industrial Science and Technology, Chiyoda-ku Tokyo 100-8921 (JP)
(72) Inventor: KOSHIYAMA, Tatsuyuki, Tokyo 103-8552 (JP); HIGASHIYAMA, Yukihiro, Tokyo 103-8552 (JP); SATO, Shun, Tsukuba-shi, Ibaraki 305-8560 (JP); MORITA, Tomotake, Tsukuba-shi, Ibaraki 305-8560 (JP); SAIKA, Azusa, Tsukuba-shi, Ibaraki 305-8560 (JP); WADA, Keisuke, Tsukuba-shi, Ibaraki 305-8560 (JP); USHIMARU, Kazunori, Tsukuba-shi, Ibaraki 305-8560 (JP)
(74) Representative: Hoefer & Partner Patentanwälte mbB
(86) International application number: PCT/JP2023/040594
(87) International publication number: WO 2024/106341

(57) **Abstract**

A microorganism according to the present disclosure is a microorganism belonging to Dirkmeia churashimaensis (accession number: NITE BP-03707, accession number: NITE BP-03708, accession number: NITE BP-03709, accession number: NITE BP-03711, or accession number: NITE BP-03712), a microorganism belonging to Aureobasidium melanogenum (accession number: NITE BP-03706), or a microorganism belonging to a species closely related to Ustilago sporoboli-indici (Ustilago sp.) (accession number: NITE BP-03710).

## Description

### TECHNICAL FIELD

The present invention relates to a novel microorganism, a novel microorganism culture or extract, and a method for producing ergothioneine.

### BACKGROUND ART

Ergothioneine is one of sulfur-containing amino acids. Ergothioneine has a higher antioxidant effect than that of vitamin E, and has attracted attention as a highly useful compound in the fields of health, beauty and the like.

For example, Patent Document 1 and Non-Patent Document 1 describe transformed filamentous fungi with enhanced ergothioneine production capability.

Non-Patent Document 2 describes a transformed microorganism of the genus Methylobacrium with enhanced ergothioneine production capability. Non-Patent Document 2 describes that microorganisms of the genera Aureobasidium and Rhodotorula have ergothioneine production capability.

Non-Patent Document 3 describes that a microorganism of the genus Pleurotus has ergothioneine production capability.

Patent Document 2 describes that microorganisms of the genera Methylobactrium and Rhodotorula have ergothioneine production capability. Patent Document 3 describes that a microorganism of the genus Moniliella has ergothioneine production capability. Patent Document 4 describes that microorganisms of the genera Dirkmeia, Papiliotrema, and Apiotrichum have ergothioneine production capability.

### CITATION LIST

### PATENT DOCUMENT

Patent Document 1: WO 2016/121285 A
Patent Document 2: WO 2016/121285 A
Patent Document 3: WO 2019/004234 A
Patent Document 4: WO 2021/140693 A

### NON-PATENT DOCUMENT

Non-Patent Document 1: S. Takusagawa, Biosci. Biotechnol. Biochem., 83, 181-184 (2019)
Non-Patent Document 2: Y. Fujitani et al., J. Biosci. Bioeng., 126, 715-722 (2018)
Non-Patent Document 3: SY. Lin, Int. J. Med. Mushrooms, 17, 749-761 (2015)

### SUMMARY OF INVENTION

### TECHNICAL PROBLEM

It is known that ergothioneine is not biosynthesized in the human body, but biosynthesized in some microorganisms. Thus, research and development on microorganisms that produce ergothioneine and modification of microorganisms to enhance the ergothioneine production are in progress, as described in the above related art documents.

Gene recombination techniques can be used to modify microorganisms to enhance the ergothioneine production. However, the ergothioneine produced by the microorganisms cannot be used in the food industry or the like. Accordingly, there is a strong desire to search for microorganisms with high ergothioneine production, which have not been subjected to gene recombination and are unmodified.

The present invention has been made in light of the above issue, and an object thereof is to provide a novel microorganism with high ergothioneine production.

### SOLUTION TO PROBLEM

As a result of screening, the present inventors have found a novel microorganism that has high ergothioneine production, and have completed the present invention.

A microorganism according to one aspect of the present invention is a microorganism belonging to Dirkmeia churashimaensis (accession number: NITE BP-03707, accession number: NITE BP-03708, accession number: NITE BP-03709, accession number: NITE BP-03711, or accession number: NITE BP-03712), a microorganism belonging to Aureobasidium melanogenum (accession number: NITE BP-03706), or a microorganism belonging to a species closely related to Ustilago sporoboli-indici (Ustilago sp.) (accession number: NITE BP-03710).

### ADVANTAGEOUS EFFECTS OF INVENTION

According to one aspect of the present invention, a microorganism having high ergothioneine production can be provided.

### BRIEF DESCRIPTION OF THE DRAWINGS

FIG. 1 is a diagram illustrating a simplified molecular phylogenetic tree based on the nucleotide sequence of the 26S rDNA-D1/D2 region of EB682 strain.
FIG. 2 is a diagram illustrating a simplified molecular phylogenetic tree based on the nucleotide sequence of the rDNA-ITS region of EB682 strain.
FIG. 3 is a diagram illustrating a simplified molecular phylogenetic tree based on the nucleotide sequence of the 26S rDNA-D1/D2 region of EC431 strain.
FIG. 4 is a diagram illustrating a simplified molecular phylogenetic tree based on the nucleotide sequence of the rDNA-ITS region of EC431 strain.
FIG. 5 is a diagram illustrating a simplified molecular phylogenetic tree based on the nucleotide sequence of the 26S rDNA-D1/D2 region of EC171 strain.
FIG. 6 is a diagram illustrating a simplified molecular phylogenetic tree based on the nucleotide sequence of the rDNA-ITS region of EC171 strain.
FIG. 7 is a diagram illustrating a simplified molecular phylogenetic tree based on the nucleotide sequence of the 26S rDNA-D1/D2 region of EC581 strain.
FIG. 8 is a diagram illustrating a simplified molecular phylogenetic tree based on the nucleotide sequence of the rDNA-ITS region of EC581 strain.
FIG. 9 is a diagram illustrating a simplified molecular phylogenetic tree based on the nucleotide sequence of the 26S rDNA-D1/D2 region of EC592 strain.
FIG. 10 is a diagram illustrating a simplified molecular phylogenetic tree based on the nucleotide sequence of the rDNA-ITS region of EC592 strain.
FIG. 11 is a diagram illustrating a simplified molecular phylogenetic tree based on the nucleotide sequence of the 26S rDNA-D1/D2 region of EB761 strain.
FIG. 12 is a diagram illustrating a simplified molecular phylogenetic tree based on the nucleotide sequence of the rDNA-ITS region of EB761 strain.
FIG. 13 is a diagram illustrating a simplified molecular phylogenetic tree based on the nucleotide sequence of the 26S rDNA-D1/D2 region of EC021 strain.
FIG. 14 is a diagram illustrating a simplified molecular phylogenetic tree based on the nucleotide sequence of the rDNA-ITS region of EC021 strain.

### DESCRIPTION OF EMBODIMENTS

In the present specification, unless otherwise specified, "A to B" representing a numerical range means "A or more (including A and more than A) and B or less (including B and less than B)".

### [Novel microorganism]

The microorganism according to one aspect of the present invention is a microorganism belonging to Dirkmeia churashimaensis or a microorganism belonging to Aureobasidium melanogenum capable of producing ergothioneine, or a microorganism belonging to the genus Ustilago capable of producing ergothioneine.

The microorganism according to one aspect of the present invention has high ergothioneine production. Ergothioneine is one of sulfur-containing amino acids and has excellent antioxidant effects. In addition, the microorganism according to one aspect of the present invention has not been modified by the gene recombination technique or the like, and thus can also be used in the food industry.

### (1. Aureobasidium melanogenum EB682)

Aureobasidium melanogenum EB682 (hereinafter it may be abbreviated as "yeast EB682") is a microorganism first isolated from hibiscus leaves as an isolation source.

The nucleotide sequences of the ribosomal RNA gene 26S rDNA-D1/D2 and ITS regions were determined. Homology search by BLAST was performed across the TechnoSuruga Laboratory microorganism identification system (TechnoSuruga Laboratory, Japan) database DB-FU13.0 and the International Nucleotide Sequence Databases (DDBJ/ENA (EMBL)/GenBank). As a result, EB682 has been attributed to Aureobasidium melanogenum.

Yeast EB682 has been deposited with NITE Patent Microorganisms Depositary (NPMD), National Institute of Technology and Evaluation (hereinafter abbreviated as "NITE") at #122, 2-5-8 Kazusakamatari, Kisarazu-shi, Chiba, Japan (Date of original deposition: August 5, 2022, accession number: NITE BP-03706).

The method for culturing Yeast EB152 may be performed in accordance with a common culture method for microorganisms of the genus Aureobasidium. The culture form is batchwise culture using a liquid medium or fed-batch culture in which a carbon source and/or an organic nitrogen source is continuously added to the culture system, and aeration agitation is desirably performed. As the culture medium, a culture medium containing carbon and nitrogen sources that are assimilable by microorganisms belonging to the genus Aureobasidium or a required nutrient source such as an inorganic salt may be used. The pH for culture is preferably 3 to 8, the culture temperature is preferably 20°C to 30°C, and the incubation time is preferably 2 to 14 days.

### (2. Ustilago sp. EC431)

Ustilago sp. EC431 (hereinafter, it may be abbreviated as "yeast EC431") is a microorganism first isolated from the fruit skin of Citrus sudachi as an isolation source.

The nucleotide sequences of the ribosomal RNA gene 26S rDNA-D1/D2 and ITS regions were determined. Homology search by BLAST was performed across the TechnoSuruga Laboratory microorganism identification system (TechnoSuruga Laboratory, Japan) database DB-FU13.0 and the International Nucleotide Sequence Databases (DDBJ/ENA (EMBL)/GenBank). As a result, it has been shown that EC431 is closely related to Ustilago sporoboli-indici.

Yeast EC431 has been deposited with NITE Patent Microorganisms Depositary (NPMD), National Institute of Technology and Evaluation (hereinafter abbreviated as "NITE") at #122, 2-5-8 Kazusakamatari, Kisarazu-shi, Chiba, Japan (Date of original deposition: August 5, 2022, accession number: NITE BP-03710).

A method for culturing yeast EC431 may be performed in accordance with a common culture method for microorganisms of the genus Ustilago. The culture form is batchwise culture using a liquid medium or fed-batch culture in which a carbon source and/or an organic nitrogen source is continuously added to the culture system, and aeration agitation is desirably performed. As the culture medium, a culture medium containing carbon and nitrogen sources that are assimilable by microorganisms belonging to the genus Ustilago or a required nutrient source such as an inorganic salt may be used. The pH for culture is preferably 3 to 8, the culture temperature is preferably 20°C to 30°C, and the incubation time is preferably 2 to 14 days.

### (3. Dirkmeia churashimaensis EC171)

Dirkmeia churashimaensis EC171 (hereinafter it may be abbreviated as "yeast EC171") is a microorganism first isolated from leaves of Brassica rapa var. perviridis as an isolation source.

The nucleotide sequences of the ribosomal RNA gene 26S rDNA-D1/D2 and ITS regions were determined. Homology search by BLAST was performed across the TechnoSuruga Laboratory microorganism identification system (TechnoSuruga Laboratory, Japan) database DB-FU13.0 and the International Nucleotide Sequence Databases (DDBJ/ENA (EMBL)/GenBank). As a result, EC171 has been attributed to Dirkmeia churashimaensis.

Yeast EC171 has been deposited with NITE Patent Microorganisms Depositary (NPMD), National Institute of Technology and Evaluation (hereinafter abbreviated as "NITE") at #122, 2-5-8 Kazusakamatari, Kisarazu-shi, Chiba, Japan (Date of original deposition: August 5, 2022, accession number: NITE BP-03709).

The method for culturing yeast EC171 may be performed in accordance with a common culture method for microorganisms of the genus Dirkmeia. The culture form is batchwise culture using a liquid medium or fed-batch culture in which a carbon source and/or an organic nitrogen source is continuously added to the culture system, and aeration agitation is desirably performed. As the medium, a medium containing carbon and nitrogen sources that are assimilable by microorganisms belonging to the genus Dirkmeia or a required nutrient source such as an inorganic salt may be used. The pH for culture is preferably 3 to 8, the culture temperature is preferably 20°C to 30°C, and the incubation time is preferably 2 to 14 days.

### (4. Dirkmeia churashimaensis EC581)

Dirkmeia churashimaensis EC581 (hereinafter it may be abbreviated as "yeast EC581") is a microorganism first isolated from kale leaves as an isolation source.

The nucleotide sequences of the ribosomal RNA gene 26S rDNA-D1/D2 and ITS regions were determined. Homology search by BLAST was performed across the TechnoSuruga Laboratory microorganism identification system (TechnoSuruga Laboratory, Japan) database DB-FU13.0 and the International Nucleotide Sequence Databases (DDBJ/ENA (EMBL)/GenBank). As a result, yeast EC581 has been attributed to Dirkmeia churashimaensis.

Yeast EC581 has been deposited with NITE Patent Microorganisms Depositary (NPMD), National Institute of Technology and Evaluation (hereinafter abbreviated as "NITE") at #122, 2-5-8 Kazusakamatari, Kisarazu-shi, Chiba, Japan (Date of original deposition: August 5, 2022, accession number: NITE BP-03711).

Yeast EC581 may be cultured in the same manner as yeast EC171.

### (5. Dirkmeia churashimaensis EC592)

Dirkmeia churashimaensis EC592 (hereinafter it may be abbreviated as "yeast EC592") is a microorganism first isolated from kale leaves as an isolation source.

The nucleotide sequences of the ribosomal RNA gene 26S rDNA-D1/D2 and ITS regions were determined. Homology search by BLAST was performed across the TechnoSuruga Laboratory microorganism identification system (TechnoSuruga Laboratory, Japan) database DB-FU13.0 and the International Nucleotide Sequence Databases (DDBJ/ENA (EMBL)/GenBank). As a result, yeast EC592 has been attributed to Dirkmeia churashimaensis.

Yeast EC592 has been deposited with NITE Patent Microorganisms Depositary (NPMD), National Institute of Technology and Evaluation (hereinafter abbreviated as "NITE") at #122, 2-5-8 Kazusakamatari, Kisarazu-shi, Chiba, Japan (Date of original deposition: August 5, 2022, accession number: NITE BP-03712).

Yeast EC592 may be cultured in the same manner as yeast EC171.

### (6. Dirkmeia churashimaensis EB761)

Dirkmeia churashimaensis EB761 (hereinafter it may be abbreviated as "yeast EB761") is a microorganism first isolated from herbaceous plant leaves as an isolation source.

The nucleotide sequences of the ribosomal RNA gene 26S rDNA-D1/D2 and ITS regions were determined. Homology search by BLAST was performed across the TechnoSuruga Laboratory microorganism identification system (TechnoSuruga Laboratory, Japan) database DB-FU13.0 and the International Nucleotide Sequence Databases (DDBJ/ENA (EMBL)/GenBank). As a result, yeast EB761 has been attributed to Dirkmeia churashimaensis.

Yeast EB761 has been deposited with NITE Patent Microorganisms Depositary (NPMD), National Institute of Technology and Evaluation (hereinafter abbreviated as "NITE") at #122, 2-5-8 Kazusakamatari, Kisarazu-shi, Chiba, Japan (Date of original deposition: August 5, 2022, accession number: NITE BP-03707).

Yeast EB761 may be cultured in the same manner as yeast EC171.

### (7. Dirkmeia churashimaensis EC021)

Dirkmeia churashimaensis EC021 (hereinafter it may be abbreviated as "yeast EC021") is a microorganism first isolated from the floral buds of Corchorus olitorius as an isolation source.

The nucleotide sequences of the ribosomal RNA gene 26S rDNA-D1/D2 and ITS regions were determined. Homology search by BLAST was performed across the TechnoSuruga Laboratory microorganism identification system (TechnoSuruga Laboratory, Japan) database DB-FU13.0 and the International Nucleotide Sequence Databases (DDBJ/ENA (EMBL)/GenBank). As a result, yeast EC021 has been attributed to Dirkmeia churashimaensis.

Yeast EC021 has been deposited with NITE Patent Microorganisms Depositary (NPMD), National Institute of Technology and Evaluation (hereinafter abbreviated as "NITE") at #122, 2-5-8 Kazusakamatari, Kisarazu-shi, Chiba, Japan (Date of original deposition: August 5, 2022, accession number: NITE BP-03708).

Yeast EC021 may be cultured in the same manner as yeast EC171.

### [Culture]

A culture according to one aspect of the present invention is a culture of yeast EB682, EC431, EC171, EC581, EC592, EB761, or EC021. The culture according to one aspect of the present invention includes, for example, culture supernatants, culture precipitates, culture medium, cultured microbial cells, and treated products of cultured microbial cells, such as crushed products of cultured microbial cells and lyophilized products of cultured microbial cells. The culture according to one aspect of the present invention contains ergothioneine.

### [Extract]

An extract according to one aspect of the present invention is an extract of yeast EB682, EC431, EC171, EC581, EC592, EB761, or EC021. As used herein, the term "extract of a microorganism" refers to a product obtained by subjecting a microorganism to extraction treatment and a product obtained by subjecting a culture of a microorganism to extraction treatment. Thus, the extract according to one aspect of the present invention can be obtained, for example, through extraction treatment of yeast EB682, EC431, EC171, EC581, EC592, EB761, or EC021, or by subjecting the culture of yeast EB682, EC431, EC171, EC581, EC592, EB761, or EC021 to extraction treatment. The extract according to one aspect of the present invention contains ergothioneine.

Examples of the extraction treatment include hot water extraction; solvent extraction by an organic solvent or the like; pressurized extraction; chemical extraction by an enzyme, a surfactant, or the like; ultrasonic extraction; alkali extraction; acid extraction; extraction by osmotic pressure; extraction by pulverization; extraction by mashing; extraction by freeze-thawing; extraction by liquid nitrogen; and extraction by high-speed agitation. From the viewpoint of exhibiting an excellent plant growth promoting effect, the extraction treatment is preferably hot water extraction. One type of extraction treatment may be performed, or two or more types of extraction treatments may be performed.

The hot water extraction is an extraction process in which an extraction target is brought into contact with or soaked in hot water for a certain time period. The temperature of water used in the hot water extraction is preferably 40°C or higher and more preferably 60°C or higher.

Examples of the extract according to one aspect of the present invention include a hot water extract; a solvent extract extracted using an organic solvent or the like; a pressurized extract; a chemical extract extracted using an enzyme, a surfactant, or the like; an ultrasonic extract; an alkali extract; an acid extract; an extract extracted using osmotic pressure; an extract extracted through pulverization; an extract extracted through mashing; an extract extracted through freeze-thawing; an extract extracted using liquid nitrogen; and an extract extracted through high-speed agitation, of a microorganism, namely yeast EB682, EC431, EC171, EC581, EC592, EB761, or EC021.

Examples of applications of the culture or extract according to one aspect of the present invention include a plant growth regulator containing the culture or extract as an active ingredient.

### [Method for producing ergothioneine]

A method for producing ergothioneine according to one aspect of the present invention includes culturing the microorganism described above to obtain a culture containing ergothioneine. In the production method, one type of microorganism may be cultured, or a plurality of types of microorganisms may be cultured.

Collection of ergothioneine from the culture containing ergothioneine may be accomplished, for example, by a common method for collecting and purifying ergothioneine from a microorganism culture. For example, the cultured microbial cells are collected by centrifugation or the like of the culture. Next, the collected microbial cells are subjected to hot water extraction or the like to obtain an extract liquid containing ergothioneine. Ergothioneine can then be collected by purifying the extract liquid. The ergothioneine production of the microorganism can be quantified, for example, by measuring the resulting extract liquid using a high-performance liquid chromatography instrument and a mass spectrometer such as LCMS.

### [Summary]

A microorganism according to one aspect of the present invention is a microorganism belonging to Dirkmeia churashimaensis (accession number: NITE BP-03707, accession number: NITE BP-03708, accession number: NITE BP-03709, accession number: NITE BP-03711, or accession number: NITE BP-03712), a microorganism belonging to Aureobasidium melanogenum (accession number: NITE BP-03706), or a microorganism belonging to a species closely related to Ustilago sporoboli-indici (Ustilago sp.) (accession number: NITE BP-03710).

A culture according to one aspect of the present invention is a culture of the microorganism described above.

An extract according to one aspect of the present invention is an extract of the microorganism described above.

A method for producing ergothioneine according to one aspect of the present invention includes culturing the microorganism described above to obtain a culture containing ergothioneine.

Embodiments of the present invention will be further described in detail using the examples below. The present invention is not limited to the examples below, and it goes without saying that various embodiments with regard to the details thereof are possible. Furthermore, the present invention is not limited to the embodiments described above, and it may be varied in various ways within the scope of the claims. Thus, an embodiment achieved by appropriately combining technical means described herein will be included in the technical scope of the present invention. In addition, the contents of all the documents referred to herein are incorporated herein by reference in their entirety.

### EXAMPLES

In the following Examples, the symbol "%" represents mass%, unless otherwise indicated.

### [Evaluation Example 1] Screening of ergothioneine-producing microorganism

### (1) Enrichment culture using isolation source collected from environment

First, microorganism sampling from environments such as plants was performed twice. As a result, a total of 150 samples (90 samples for the first time and 60 samples for the second time) were collected.

Then, the samples collected were each immersed in a 15-mL plastic tube containing 2 mL of a screening medium, and cultured at 200 rpm and 25°C for 3 to 7 days. The screening medium used was a YM medium containing an antibiotic. Specifically, a medium containing 1% glucose, 0.5% peptone, 0.3% yeast extract, 0.3% malt extract, 0.01% streptomycin sulfate, and 0.005% chloramphenicol was used.

Then, 126 samples (70 samples for the first time and 56 samples for the second time) in which the medium was visually observed to be cloudy (microorganisms proliferated) were selected.

### (2) Selection of samples with oxidative stress load

Culture solutions of the 126 samples selected in (1) above were each diluted 100 or 10000 times in a YM medium. The diluted culture solution was applied to a YM agar medium and a YM agar medium added with 3 mM H₂O₂ (hereinafter abbreviated as H₂O₂-containing YM agar medium), and cultured at 25°C for 2 to 7 days.

The number of colonies having grown on the YM agar medium and the number of colonies having grown on the H₂O₂-containing YM agar medium were counted. Then, 112 samples (69 samples for the first time and 43 samples for the second time) in which colonies had grown on both the YM agar medium and the H₂O₂-containing YM agar medium were selected.

In addition, for the colonies having grown on the agar medium in the selected 112 samples, the morphology and color were visually observed, and 181 yeast-like colonies of different types (106 colonies for the first time and 75 colonies for the second time) were selected.

### (3) Culture of selected colonies in 96 wells

The 181 colonies selected in (2) above were inoculated into 96 well plates containing 1 mL of a YM medium, and cultured at 1600 rpm and 25°C for 3 to 4 days. After culturing, the collected culture solutions were centrifuged at 2000 rpm and 4°C for 10 minutes. The cell pellets obtained by centrifugation were washed with pure 1 mL and centrifuged again.

To the cell pellets obtained by centrifugation, 0.1 mL of pure water was added to suspend the pellets therein. The resulting suspensions were heated at 96°C for 10 minutes to extract the intracellular components. The extracted intracellular components were then centrifuged to remove microbial cell residues, thereby obtaining extract liquids.

### (4) Quantitative analysis of ergothioneine in extract liquid by LCMS

A mixed solution of 0.15 mL of each of the extract liquids obtained in (3) above and 0.35 mL of acetonitrile was filtered through a 0.45-µm PVDF filter. The resulting filtrate was used as a sample for LCMS measurement.

LCMS-2020, available from Shimadzu Corporation, was used for LCMS analysis. In addition, an Asahipak NH2P-40 2D+ guard column, available from SHODEX, was used as the column for LC. A mixed solution of 10 mM ammonium formate and acetonitrile (10 mM ammonium formate/acetonitrile = 30/70 (v/v)) was used as the mobile phase for LC. The flow rate was set to 0.1 mL/min, and analysis was performed at 25°C.

In MS detection, ionization was performed in DUIS mode for performing ESI ionization and APCI ionization simultaneously. Detection was also performed in SIM mode of m/z = 230 (+) in which ergothioneine could be detected.

As a result of analyzing the extract liquids of the 181 colonies selected in (2) above, 22 colonies with high ergothioneine production (7 colonies for the first time and 15 colonies for the second time) were selected.

### (5) Scale-up culture of ergothioneine-producing microorganism in flask

The 22 colonies selected in (4) above were each inoculated into a 300-mL flask containing 50 mL of a YM medium, and cultured at 200 rpm and 25°C for 7 days (n = 1).

The culture solutions on Days 3 to 7 were collected as appropriate. As in (3) above, after centrifugation and washing of the microbial cells, extract liquids were collected by hot water extraction.

The resulting extract liquids were analyzed by LCMS in the same manner as in (4) above to select seven strains (EB682, EC431, EC171, EC581, EC592, EB761, and EC021) with high ergothioneine production.

### [Evaluation Example 2] Measurement of ergothioneine production

EB682, EC431, EC171, EC581, and EC592 were each inoculated into a 300-mL flask containing 50 mL of a YM medium, and cultured at 200 rpm and 25°C for 5 days (n = 3). The ergothioneine (EGT) productions on Day 5 were then measured by LCMS.

The extract of each microorganism was obtained by aerobically culturing each microorganism at 25°C for 5 days using a 5-L jar fermenter containing 2 L of a YM medium, and subjecting dried microbial cells after culturing to hot water extraction. Then, the EGT amount (mg/L) in the extract was measured by LCMS.

The ergothioneine production, production rate, and EGT amount of each strain are shown in Tables 1 and 2. The EGT production (mg/L-culture solution) in Table 1 is the EGT production on Day 5 per 1 L of culture solution. EGT production rate (mg/L/d) is the EGT production per day (mg/L). The EGT production (mg/g-dried microbial cell) in Table 2 is the EGT production per 1 g of dried microbial cell.

Tables 3 and 4 show the productions and production rates of known microorganisms. In Table 3, the EGT production of Aspergillus oryzae NSAR1 is the EGT production per 1 kg of culture solution, and the EGT production rate is the EGT production per day (mg/kg). "-" in Tables 3 and 4 indicates that the measurement was not performed.

### [Table 1]

**Table 1**

| Strain | Microorganism name | EGT production | EGT production rate |
|---|---|---|---|
| | | (mg/L-culture solution) | (mg/L/d) |
| EB682 | *Aureobasidium melanogenum* | 37.0 ± 4.1 | 7.4 |
| EC431 | *Ustilago sp.* | 49.9 ± 2.1 | 10.0 |
| EC171 | *Dirkmeia churashimaensis* | 53.8 ± 3.6 | 10.8 |
| EC581 | *Dirkmeia churashimaensis* | 58.9 ± 2.0 | 11.8 |
| EC592 | *Dirkmeia churashimaensis* | 53.5 ± 1.4 | 10.7 |

### [Table 2]

**Table 2**

| Strain | Microorganism name | EGT production | EGT amount in extract |
|---|---|---|---|
| | | (mg/g-_{dried microbial cell}) | (mg/L) |
| EB682 | *Aureobasidium melanogenum* | 10.9 | 110 |
| EC431 | *Ustilago sp.* | 12.2 | 496 |
| EC171 | *Dirkmeia churashimaensis* | 12.8 | 565 |
| EC581 | *Dirkmeia churashimaensis* | 15.1 | 460 |
| EC592 | *Dirkmeia churashimaensis* | 13.8 | 462 |

### [Table 3]

**Table 3**

| Microorganism name | EGT production | EGT production rate |
|---|---|---|
| | (mg/L-_{culture solution}) | (mg/L/d) |
| *Aureobasidium pullulans* kz25 | 14 | 2 |
| *Aspergillus oryzae* NSAR1 | 11.5 (mg/kg) | 2.3 (mg/kg/d) |
| *Pleurotus citrinopileatus* | 13 to 98 | 0.8 to 6.1 |
| *Methylobacterium aquaticum* 22A | 12.2 | 1.7 |
| *Dirkmeia churashimaensis* S111 | 29.5 ± 3.5 | 5.9 |
| *Ustilago maydis* UM521 | 9.1 | 3.0 |

### [Table 4]

**Table 4**

| Microorganism name | EGT production | Reference |
|---|---|---|
| | (mg/g-_{dried microbial cell}) | |
| *Aureobasidium pullulans* kz25 | 1 | J Biosci Bioeng 126 (2018) 715 |
| *Aspergillus oryzae* NSAR1 | - | Biosci Biotechnol Biochem 83 (2019) 181 |
| *Pleurotus citrinopileatus* | - | I J Med Mushroom 17 (2015) 749 |
| *Methylobacterium aquaticum* 22A | - | Biosci Biotechnol Biochem 83 (2019) 181 |
| *Dirkmeia churashimaensis* S111 | 6.4 | WO2021/140693 |
| *Ustilago maydis* UM521 | - | JP2021-141826A |

As shown in Tables 1 and 2, it was found that Aureobasidium melanogenum EB682 had high EGT production compared to Aureobasidium pullulans kz25. It was found that Ustilago sp. EC431 had high EGT production compared to Ustilago maydis UM521. It was found that Dirkmeia churashimaensis EC171, Dirkmeia churashimaensis EC581, and Dirkmeia churashimaensis EC592 had high EGT production compared to Dirkmeia churashimaensis S111. It was also confirmed that all of the extracts of the five strains evaluated in Evaluation Example 2 contained EGT abundantly.

### [Evaluation Example 3] Measurement of ergothioneine production

For EB761 and EC021, the EGT production on Day 5 was measured by LCMS in the same manner as in Evaluation Example 2. The EGT amount in the extract of each microorganism was also measured in the same manner as in Evaluation Example 2. The measurement results are shown in Table 5.

### [Table 5]

**Table 5**

| Strain | Microorganism name | EGT production | EGT amount in extract |
|---|---|---|---|
| | | (mg/L-_{dried microbial cell}) | (mg/L) |
| EB761 | *Dirkmeia churashimaensis* | 8.1 | 510 |
| EC021 | *Dirkmeia churashimaensis* | 8.9 | 378 |

As shown in Tables 4 and 5, it was found that Dirkmeia churashimaensis EB761 and Dirkmeia churashimaensis EC021 each had high EGT production compared to Dirkmeia churashimaensis S111. It was also confirmed that both the extracts of the two strains evaluated in Evaluation Example 3 contained EGT abundantly.

### [Evaluation Example 4] Identification of microorganism

Estimation of the classification groups to which the selected seven strains were attributed was performed by analysis of the nucleotide sequences of the ribosomal RNA gene 26S rDNA-D1/D2 and ITS regions.

### (Molecular phylogenetic position and morphological properties of EB682 strain)

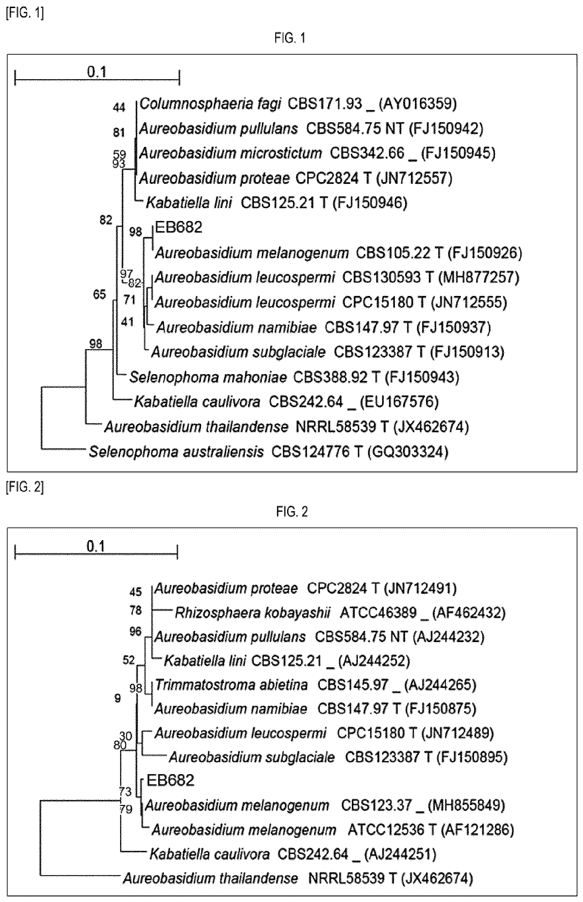

As a result of homology search by BLAST on DB-FU and International Nucleotide Sequence Databases using the microbial identification system "ENKI", the nucleotide sequence (SEQ ID NO: 1) of the 26S rDNA-D1/D2 region of EB682 strain showed 100% identity to a plurality of nucleotide sequences of Aureobasidium melanogenum, which is a type of ascomycete yeasts (Tables 6 and 7). In the molecular phylogenetic tree (FIG. 1) analyzed based on the nucleotide sequence obtained by homology search on DB-FU, EB682 strain showed the same molecular phylogenetic position as that of Aureobasidium melanogenum CBS105. 22T (accession number FJ150926).

Table 6 shows the results of a search by BLAST on DB-FU, that is, the nucleotide sequence analysis data for the 26S rDNA-D1/D2 region found in the top 30 homology scores. The data with * is sequence data subjected to simplified molecular phylogenetic analysis.

Table 7 shows the results of a search by BLAST on International Nucleotide Sequence Databases, that is, the nucleotide sequence analysis data for the 26S rDNA-D1/D2 region found in the top 30 homology scores. The data with * is sequence data subjected to simplified molecular phylogenetic analysis. Aureobasidium pullulans var. melanigenum is considered to correspond to the current name, Aureobasidium melanogenum.

FIG. 1 shows a simplified molecular phylogenetic tree based on the nucleotide sequence of the 26S rDNA-D1/D2 region of EB682 strain. The upper left line indicates the scale bar. The numbers positioned at the branches of the phylogenetic branches indicate bootstrap values. "T" at the end of a strain name indicates the type strain of the species, and "NT" indicates the new type strain of the species.

### [Table 6]

**Table 6**

| Registration name | Strain name | Accession No. | Homology rate |
|---|---|---|---|
| *Aureobasidium melanogenum ** | CBS105.22 | FJ150926 | 571/571 (100.0%) |
| *Aureobasidium leucospermi ** | CBS130593 | MH877257 | 564/571 (98.8%) |
| *Aureobasidium leucospermi ** | CPC15180 | JN712555 | 563/571 (98.6%) |
| *Aureobasidium namibiae ** | CBS147.97 | FJ150937 | 563/571 (98.6%) |
| *Aureobasidium subglaciale ** | CBS123387 | FJ150913 | 565/572 (98.8%) |
| *Selenophoma mahoniae ** | CBS388.92 | FJ150943 | 558/571 (97.7%) |
| *Columnosphaeria fagi ** | CBS171.93 | AY016359 | 556/571 (97.4%) |
| *Aureobasidium pullulans ** | CBS584.75 | FJ150942 | 556/571 (97.4%) |
| *Aureobasidium microstictum ** | CBS342.66 | FJ150945 | 556/571 (97.4%) |
| *Aureobasidium proteae ** | CPC2824 | JN712557 | 550/565 (97.3%) |
| *Kabatiella lini ** | CBS125.21 | FJ150946 | 553/571 (96.8%) |
| *Kabatiella caulivora ** | CBS242.64 | EU167576 | 550/571 (96.3%) |
| *Aureobasidium thailandense ** | NRRL58539 | JX462674 | 543/570 (95.3%) |
| *Selenophoma australiensis ** | CBS124776 | GQ303324 | 536/573 (93.5%) |
| *Hormonema viticola* | L9D-17 | KF201298 | 530/571 (92.8%) |
| *Pringsheimia smilacis* | CBS873.71 | FJ150970 | 528/571 (92.5%) |
| *Dothiora cannabinae* | CBS737.71 | DQ470984 | 523/563 (92.9%) |
| *Dothidea sambuci* | CBS198.58 | AY930109 | 528/571 (92.5%) |
| *Dothidea sambuci* | CBS197.58 | AY930108 | 528/571 (92.5%) |
| *Dothidea sambuci* | DAOM231303 | NG 027611 | 527/571 (92.3%) |
| *Stylodothis puccinioides* | CBS193.58 | NG 027594 | 525/571 (91.9%) |
| *Dothidea ribesia* | CBS195.58 | AY016360 | 525/571 (91.9%) |
| *Coniozyma leucospermi* | CBS111289 | EU552113 | 524/571 (91.8%) |
| *Dothidea insculpta* | CBS189.58 | DQ247802 | 518/564 (91.8%) |
| *Dothidea insculpta* | CBS189.58 | NG 027643 | 518/564 (91.8%) |
| *Sydowia polyspora* | CBS16.29 | DQ678058 | 522/571 (91.4%) |
| *Dothiora elliptica* | CBS736.71 | GU301811 | 508/549 (92.5%) |
| *Delphinella strobiligena* | CBS735.71 | DQ470977 | 515/563 (91.5%) |
| *Sydowia polyspora* | CBS750.71 | FJ150912 | 499/548 (91.1%) |
| *Phaeocryptopus nudus* | CBS268.37 | GU301856 | 493/538 (91.6%) |

### [Table 7]

**Table 7**

| Registration name | Strain name | Accession No. | Homology rate |
|---|---|---|---|
| *Aureobasidium melanogenum* | SDBR-S2-07 | MT623572 | 571/571 (100.0%) |
| *Aureobasidium melanogenum* | SDBR-S1-10 | MT613410 | 571/571 (100.0%) |
| *Aureobasidium pullulans* ^{a} | UBOCC-A-118066 | MT237304 | 571/571 (100.0%) |
| *Aureobasidium pullulans* ^{a} | GXL-1 | MT083975 | 571/571 (100.0%) |
| *Aureobasidium melanogenum* | - | MN602153 | 571/571 (100.0%) |
| *Aureobasidium melanogenum* | - | MN602152 | 571/571 (100.0%) |
| *Aureobasidium melanogenum* | - | MN602151 | 571/571 (100.0%) |
| *Aureobasidium melanogenum* | - | MN602150 | 571/571 (100.0%) |
| *Aureobasidium melanogenum* | - | MN602149 | 571/571 (100.0%) |
| *Aureobasidium melanogenum* | - | MN602148 | 571/571 (100.0%) |
| *Aureobasidium pullulans* ^{a} | Y.H. Yeh V0313 | MH160817 | 571/571 (100.0%) |
| *Aureobasidium melanogenum* | CBS125735 | MH875142 | 571/571 (100.0%) |
| *Aureobasidium pullulans* ^{a} | CBS 110.67 | MH870594 | 571/571 (100.0%) |
| *Aureobasidium melanogenum* | CBS 105.22 | MH866219 | 571/571 (100.0%) |
| *Aureobasidium melanogenum* | LUM015 | MH892855 | 571/571 (100.0%) |
| *Aureobasidium melanogenum* | 7-1 | MF939078 | 571/571 (100.0%) |
| *Aureobasidium melanogenum* | 11-1 | MH019967 | 571/571 (100.0%) |
| *Aureobasidium pullulans* ^{a} | KUC1512 | KX893323 | 571/571 (100.0%) |
| *Aureobasidium melanogenum* | 88 | KU962119 | 571/571 (100.0%) |
| *Aureobasidium melanogenum* | ATCC 9348 | KU933415 | 571/571 (100.0%) |
| *Aureobasidium pullulans* ^{a} | DMKU-SE136 | LC177047 | 571/571 (100.0%) |
| Aureobasidium sp. | - | MT863787 | 571/571 (100.0%) |
| *Aureobasidium pullulans* ^{a} | LM021 | AB617922 | 571/571 (100.0%) |
| *Aureobasidium pullulans* ^{a} | LM027 | AB617928 | 571/571 (100.0%) |
| *Aureobasidium pullulans* ^{a} | LM020 | AB617921 | 571/571 (100.0%) |
| *Aureobasidium sp.* | RBF-6C1 | FN665418 | 571/571 (100.0%) |
| *Aureobasidium sp.* | RBF-3B2 | FN665417 | 571/571 (100.0%) |
| *Aureobasidium pullulans* ^{a} | SN22 | FJ515219 | 571/571 (100.0%) |
| *Aureobasidium pullulans* ^{a} | CRUB 1145 | EF595769 | 571/571 (100.0%) |
| *Aureobasidium melanogenum* | CBS 105.22 | NG 056960 | 571/571 (100.0%) |

As a result of homology search by BLAST on DB-FU and International Nucleotide Sequence Databases using the microbial identification system "ENKI", the nucleotide sequence (SEQ ID NO: 2) of the rDNA-ITS region of EB682 strain showed 99.8 to 100% identity to a plurality of nucleotide sequences of Aureobasidium melanogenum, which is a type of ascomycete yeasts (Tables 8 and 9). In the molecular phylogenetic tree (FIG. 2) analyzed based on the nucleotide sequence obtained by homology search on DB-FU, EB682 strain formed a cluster with a plurality of nucleotide sequences of Aureobasidium melanogenum.

Table 8 shows the results of a search by BLAST on DB-FU, that is, the nucleotide sequence analysis data for the rDNA-ITS region found in the top 30 homology scores. The data with * is sequence data subjected to simplified molecular phylogenetic analysis.

Table 9 shows the results of a search by BLAST on International Nucleotide Sequence Databases, that is, the nucleotide sequence analysis data for the rDNA-ITS region found in the top 30 homology scores. The data with * is sequence data subjected to simplified molecular phylogenetic analysis. Aureobasidium pullulans var. melanigenum is considered to correspond to the current name, Aureobasidium melanogenum.

FIG. 2 shows a simplified molecular phylogenetic tree based on the nucleotide sequence of the rDNA-ITS region of EB682 strain. The upper left line indicates the scale bar. The numbers positioned at the branches of the phylogenetic branches indicate bootstrap values. "T" at the end of a strain name indicates the type strain of the species, and "NT" indicates the new type strain of the species.

### [Table 8]

**Table 8**

| Registration name | Strain name | Accession No. | Homology rate |
|---|---|---|---|
| *Aureobasidium melanogenum ** | CBS123.37 | MH855849 | 562/563 (99.8%) |
| *Aureobasidium leucospermi ** | CPC15180 | JN712489 | 539/545 (98.9%) |
| *Aureobasidium proteae* * | CPC2824 | JN712491 | 536/543 (98.7%) |
| *Rhizosphaera kobayashii* * | ATCC46389 | AF462432 | 543/564 (96.3%) |
| *Aureobasidium melanogenum* * | ATCC12536 | AF121286 | 505/508 (99.4%) |
| *Trimmatostroma abietina ** | CBS145.97 | AJ244265 | 501/507 (98.8%) |
| *Aureobasidium pullulans ** | CBS584.75 | AJ244232 | 499/506 (98.6%) |
| *Kabatiella lini ** | CBS125.21 | AJ244252 | 496/508 (97.6%) |
| *Aureobasidium thailandense ** | NRRL58539 | JX462674 | 536/573 (93.5%) |
| *Aureobasidium namibiae ** | CBS147.97 | FJ150875 | 481/486 (99.0%) |
| *Kabatiella caulivora ** | CBS242.64 | AJ244251 | 493/508 (97.0%) |
| *Aureobasidium subglaciale ** | CBS123387 | FJ150895 | 466/474 (98.3%) |
| *Rhizosphaera kalkhoffii* | ATCC46388 | AY183366 | 505/577 (87.5%) |
| *Rhizosphaera macrospora* | ATCC4636 | AF462431 | 507/580 (87.4%) |
| *Rhizosphaera pini* | ATCC46387 | AY183365 | 504/578 (87.2%) |
| *Kabatina thujae* | CBS238.66 | AF013226 | 497/576 (86.3%) |
| *Rhizosphaera oudemansii* | ATCC46390 | AF462430 | 506/580 (87.2%) |
| *Coniozyma leucospermi* | CBS111289 | EU552113 | 498/580 (85.9%) |
| *Cylindroseptoria ceratoniae* | CBS477.69 | KF251151 | 481/558 (86.2%) |
| *Hormonema viticola* | L9D-17 | KP641179 | 490/578 (84.8%) |
| *Pringsheimia smilacis* | CBS873.71 | AJ244257 | 462/530 (87.2%) |
| *Celosporium larixicola* | L3-1 (UAMH11008) | FJ997287 | 466/545 (85.5%) |
| *Sydowia polyspora* | CBS215.50 | AJ244242 | 454/530 (85.7%) |
| *Dothidea sambuci* | CBS198.58 | AY930109 | 445/519 (85.7%) |
| *Dothidea sambuci* | CBS197.58 | AY930108 | 445/519 (85.7%) |
| *Sydowia polyspora* | CBS544.95 | AY293068 | 463/548 (84.5%) |
| *Coniothyrium leucospermi* | CBS114035 | AY720707 | 453/535 (84.7%) |
| *Dothidea sambuci* | DAOM231303 | NR 111220 | 431/505 (85.3%) |
| *Dothidea insculpta* | CBS189.58 | AF027764 | 451/535 (84.3%) |
| *Hormonema macrosporum* | CBS536.94 | AJ244247 | 442/525 (84.2%) |

### [Table 9]

**Table 9**

| Registration name | Strain name | Accession No. | Homology rate |
|---|---|---|---|
| *Aureobasidium melanogenum* | 7-1 | MF939079 | 563/563 (100.0%) |
| aff. *Aureobasidium sp.* | - | KT150681 | 563/563 (100.0%) |
| aff. *Aureobasidium sp.* | - | KT150632 | 563/563 (100.0%) |
| aff. *Aureobasidium sp.* | - | KT150628 | 563/563 (100.0%) |
| uncultured eukaryote | - | KJ180768 | 563/563 (100.0%) |
| uncultured eukaryote | - | GU941387 | 563/563 (100.0%) |
| *Aureobasidium pullulans* ^{a} | - | AY225166 | 563/563 (100.0%) |
| *Aureobasidium pullulans* ^{a} | - | JQ235063 | 562/562 (100.0%) |
| *Aureobasidium pullulans* ^{a} | ZD-3D | JF422784 | 562/562 (100.0%) |
| *Aureobasidium melanogenum* | Y. H. Yeh 10527 | MK336633 | 562/563 (99.8%) |
| *Aureobasidium melanogenum* | CBS 123.37 | MH855849 | 562/563 (99.8%) |
| *Aureobasidium pullulans* ^{a} | UBOCC-A-111234 | MH102060 | 562/563 (99.8%) |
| *Aureobasidium pullulans* ^{a} | IFM 64153 | LC317470 | 562/563 (99.8%) |
| *Aureobasidium pullulans* ^{a} | IFM 63511 | LC317468 | 562/563 (99.8%) |
| *Aureobasidium melanogenum* | KAS5840 | KY659501 | 562/563 (99.8%) |
| *Aureobasidium melanogenum* | KUC3075 | KY294711 | 562/563 (99.8%) |
| *Aureobasidium melanogenum* | KUC3073 | KY294710 | 562/563 (99.8%) |
| *Aureobasidium melanogenum* | KUC1738 | KY294709 | 562/563 (99.8%) |
| *Aureobasidium melanogenum* | KUC1632 | KY294708 | 562/563 (99.8%) |
| *Aureobasidium pullulans* ^{a} | kz4 | LC277145 | 562/563 (99.8%) |
| *Aureobasidium pullulans* ^{a} | JCM 22445 | LC228678 | 562/563 (99.8%) |
| uncultured fungus | - | KX515375 | 562/563 (99.8%) |
| uncultured fungus | - | KX515060 | 562/563 (99.8%) |
| uncultured fungus | - | KX514939 | 562/563 (99.8%) |
| *Aureobasidium melanogenum* | ATCC 9348 | KU933441 | 562/563 (99.8%) |
| aff. *Aureobasidium sp.* | - | KT150660 | 562/563 (99.8%) |
| uncultured fungus | - | KU164701 | 562/563 (99.8%) |
| uncultured fungus | - | KU164697 | 562/563 (99.8%) |
| uncultured fungus | - | KU164695 | 562/563 (99.8%) |
| *Aureobasidium melanogenum* | - | MT781960 | 562/563 (99.8%) |

Based on the above, EB682 strain was identified as Aureobasidium melanogenum in the results of nucleotide sequence analysis for the 26S rDNA-D1/D2 region and the rDNA-ITS region.

The morphological properties of EB682 strain were examined through observation of the nature and morphology of the colonies. As a result of culturing on a **YM** plate medium for 2 days, cream to light brown-colored colony nature with a moist and mycelial surface was exhibited. On Day 3, the formation of colorless, thin-walled, wide-oval to lemon-shaped yeast-like budding cells was confirmed. In addition, it was observed that a wide-oval to lemon-shaped, workless as one cell, and smooth blastoconidium was formed from a short protruding structure on the vegetative hyphae.

Based on the molecular phylogenetic position and morphological properties as well as the measurement of ergothioneine production, EB682 strain was determined to be a novel microorganism attributed to Aureobasidium melanogenum.

### (Molecular phylogenetic position and morphological properties of EC431 strain)

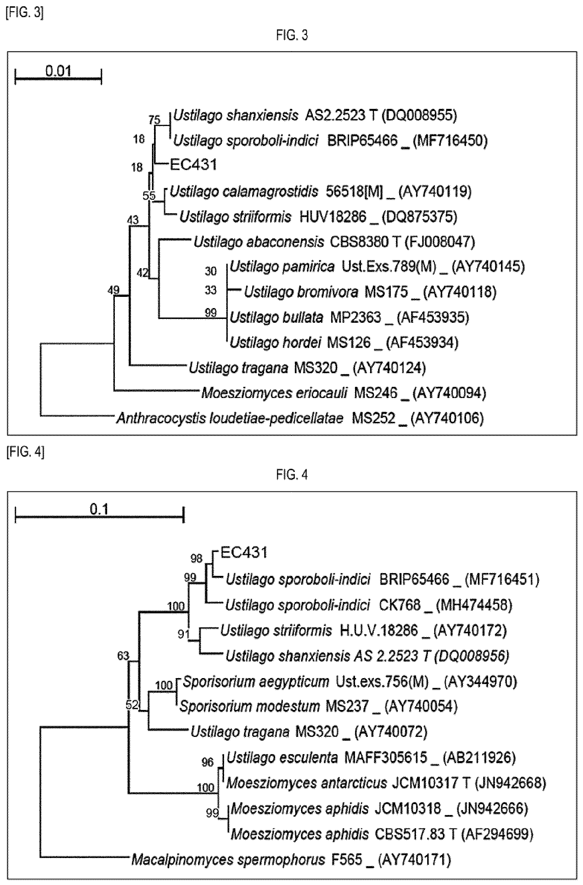

As a result of homology search by BLAST on DB-FU and International Nucleotide Sequence Databases using the microbial identification system "ENKI", the nucleotide sequence (SEQ ID NO: 3) of the 26S rDNA-D1/D2 region of EC431 strain showed 99.7% identity to a plurality of nucleotide sequences of Ustilago shanxiensis, Ustilago calamagrostidis, and Ustilago sporoboli-indici, which are types of basidiomycetes (basidiomycete yeast) (Tables 10 and 11). In the molecular phylogenetic tree (FIG. 3) analyzed based on the nucleotide sequence obtained by homology search on DB-FU and International Nucleotide Sequence Databases, EC431 strain formed a single phylogenetic branch in the phylogenetic group composed of the genus Ustilago.

Table 10 shows the results of a search by BLAST on DB-FU, that is, the nucleotide sequence analysis data for the 26S rDNA-D1/D2 region found in the top 30 homology scores. The data with * is sequence data subjected to simplified molecular phylogenetic analysis.

Table 11 shows the results of a search by BLAST on International Nucleotide Sequence Databases, that is, the nucleotide sequence analysis data for the 26S rDNA-D1/D2 region found in the top 30 homology scores. The data with * is sequence data subjected to simplified molecular phylogenetic analysis.

FIG. 3 shows a simplified molecular phylogenetic tree based on the nucleotide sequence of the 26S rDNA-D1/D2 region of EC431 strain. The upper left line indicates the scale bar. The numbers positioned at the branches of the phylogenetic branches indicate bootstrap values. "T" at the end of a strain name indicates the type strain of the species.

### [Table 10]

**Table 10**

| Registration name | Strain name | Accession No. | Homology rate |
|---|---|---|---|
| *Ustilago shanxiensis ** | AS2.2523 | DQ008955 | 603/605 (99.7%) |
| *Ustilago calamagrostidis ** | 56518[M] | AY740119 | 603/605 (99.7%) |
| *Ustilago striiformis ** | HUV18286 | DQ875375 | 602/605 (99.5%) |
| *Ustilago abaconensis ** | CBS8380 | FJ008047 | 601/605 (99.3%) |
| *Ustilago tragana ** | MS320 | AY740124 | 598/605 (98.8%) |
| *Ustilago pamirica* * | Ust.Exs.789(M) | AY740145 | 598/605 (98.8%) |
| *Ustilago hordei* * | MS126 | AF453934 | 598/605 (98.8%) |
| *Ustilago bullata* * | M P2363 | AF453935 | 598/605 (98.8%) |
| *Ustilago bromivora ** | MS175 | AY740118 | 597/605 (98.7%) |
| *Moesziomyces eriocauli ** | MS246 | AY740094 | 597/605 (98.7%) |
| *Macalpinomyces eragrostiellae* | Ust.Exs.960(M) | AY740089 | 597/605 (98.7%) |
| *Anthracocystis elionuri* | MP2601 | AY740157 | 597/605 (98.7%) |
| *Ustilago trichophora* | MS37 | AY740148 | 596/605 (98.5%) |
| *Ustilago trichophora* | MS339 | AY740125 | 596/605 (98.5%) |
| *Ustilago cynodontis* | MP1838 | AF009881 | 596/605 (98.5%) |
| *Anthracocystis chrysopogonis* | MS135 | AY740131 | 596/605 (98.5%) |
| *Ustilago nuda* | HUV17782 | JN367334 | 596/604 (98.7%) |
| *Erratomyces patelii* | CBS669.70 | DQ094784 | 590/596 (99.0%) |
| *Ustilago trichophora* | MP1898 | AJ236141 | 595/605 (98.3%) |
| *Ustilago sparsa* | KVU892 | JN367335 | 589/595 (99.0%) |
| *Ustilago echinata* | Ust.Exs.540 | AY740144 | 595/605 (98.3%) |
| *Sporisorium andropogonis* | MS283 | AY740095 | 595/605 (98.3%) |
| *Macalpinomyces loudetiae* | MS250 | AY740151 | 595/605 (98.3%) |
| *Dirkmeia churashimaensis* | DMKU-CE131 | LC178822 | 595/605 (98.3%) |
| *Anthracocystis apludae-aristatae* | MS287 | AY740098 | 595/605 (98.3%) |
| *Anthracocystis themedae-arguentis* | Ust.Exs.855 | AY740140 | 595/605 (98.3%) |
| *Anthracocystis ovaria* | MP1871 | AJ236137 | 598/608 (98.4%) |
| *Anthracocystis loudetiae-pedicellatae ** | MS252 | AY740106 | 596/606 (98.3%) |
| *Ustilago xerochloae* | Ust.Exs.1000(M) | AY740150 | 590/598 (98.7%) |
| *Anthracocystis pampara* | JCM2007 | KP322980 | 597/607 (98.4%) |

### [Table 11]

**Table 11**

| Registration name | Strain name | Accession No. | Homology rate |
|---|---|---|---|
| *Ustilago sporoboli-indici* * | BRIP65466 | MF716450 | 603/605 (99.7%) |
| *Ustilago sporoboli-indici* | BRIP39706 | MF716449 | 603/605 (99.7%) |
| *Ustilago shanxiensis* | - | NG058425 | 603/605 (99.7%) |
| *Ustilago shanxiensis* | - | FJ515237 | 603/605 (99.7%) |
| *Ustilago shanxiensis* | AS 2.2523 (ex-type) | DQ008955 | 603/605 (99.7%) |
| *Ustilago calamagrostidis* | - | AY740119 | 603/605 (99.7%) |
| *Ustilago striiformis* | - | KF381043 | 602/605 (99.5%) |
| *Ustilago striiformis* | - | KF381042 | 602/605 (99.5%) |
| *Ustilago striiformis* | - | KF381041 | 602/605 (99.5%) |
| *Ustilago striiformis* | - | KF381029 | 602/605 (99.5%) |
| *Ustilago striiformis* | - | DQ875375 | 602/605 (99.5%) |
| *Ustilago striiformis* | - | AY740172 | 602/605 (99.5%) |
| uncultured fungus | - | JQ311706 | 601/604 (99.5%) |
| *Ustilago striiformis* | - | KF381034 | 601/605 (99.3%) |
| uncultured fungus | - | JQ310924 | 600/604 (99.3%) |
| *Ustilago abaconensis* | CBS 8380 | NG057804 | 601/605 (99.3%) |
| *Ustilago striiformis* | - | KF381033 | 600/605 (99.2%) |
| *Ustilago sparsa* | CBS131471 | MH877397 | 599/605 (99.0%) |
| *Ustilago sp.* | SMN03 | KF922222 | 599/605 (99.0%) |
| *Ustilago striiformis* | - | KF381046 | 594/597 (99.5%) |
| *Ustilago sparsa* | - | MT279744 | 598/604 (99.0%) |
| *Ustilago shanxiensis* | TO 1063 | MW057410 | 589/589 (100.0%) |
| *Ustilago levis x avenae* | CBS396.36 | MH867342 | 598/605 (98.8%) |
| *Ustilago levis* | CBS393.36 | MH867340 | 598/605 (98.8%) |
| *Ustilago levis* | CBS 345.32 | MH866806 | 598/605 (98.8%) |
| *Ustilago hordei* | CBS343.32 | MH866805 | 598/605 (98.8%) |
| *Ustilago hordei* | CBS342.32 | MH866804 | 598/605 (98.8%) |
| *Ustilago bullata* | CBS201.26 | MH866382 | 598/605 (98.8%) |
| *Anthracocystis prodzinskae* | UFT5852 | KX588719 | 598/605 (98.8%) |
| *Ustilago bromivora* | UB2112 | LT558136 | 598/605 (98.8%) |

As a result of homology search by BLAST on DB-FU and International Nucleotide Sequence Databases using the microbial identification system "ENKI", the nucleotide sequence (SEQ ID NO: 4) of the rDNA-ITS region of EC431 strain showed 95.5 to 97.9% identity to a plurality of nucleotide sequences of Ustilago sporoboli-indici, which is a type of basidiomycetes (basidiomycete yeast) (Tables 12 and 13). In the molecular phylogenetic tree (FIG. 4) analyzed based on the nucleotide sequence obtained by homology search on DB-FU and International Nucleotide Sequence Databases, EC431 strain is included in the phylogenetic group composed of the genus Ustilago and formed a cluster supported with a plurality of nucleotide sequences of Ustilago sporoboli-indici with a bootstrap value of 99%. However, since the nucleotide sequences of the rDNA-ITS region of SIID35092-03 and Ustilago sporoboli-indici differed by 13 or more of nucleotides, it was difficult to determine the attribution at the species level, and it was considered to be appropriate as attributed to Ustilago sp. closely related to Ustilago sporoboli-indici.

Table 12 shows the results of a search by BLAST on DB-FU, that is, the nucleotide sequence analysis data for the rDNA-ITS region found in the top 30 homology scores. The data with * is sequence data subjected to simplified molecular phylogenetic analysis.

Table 13 shows the results of a search by BLAST on International Nucleotide Sequence Databases, that is, the nucleotide sequence analysis data for the rDNA-ITS region found in the top 30 homology scores. The data with * is sequence data subjected to simplified molecular phylogenetic analysis.

FIG. 4 shows a simplified molecular phylogenetic tree based on the nucleotide sequence of the rDNA-ITS region of EC431 strain. The upper left line indicates the scale bar. The numbers positioned at the branches of the phylogenetic branches indicate bootstrap values. "T" at the end of a strain name indicates the type strain of the species.

### [Table 12]

**Table 12**

| Registration name | Strain name | Accession No. | Homology rate |
|---|---|---|---|
| *Sporisorium aegypticum ** | Ust.exs.756(M) | AY344970 | 658/758 (86.8%) |
| *Sporisorium modestum* * | MS237 | AY740054 | 647/744 (87.0%) |
| *Ustilago esculenta ** | MAFF305615 | AB211926 | 654/770 (84.9%) |
| *Moesziomyces antarcticus ** | JCM10317 | JN942668 | 654/770 (84.9%) |
| *Ustilago tragana ** | MS320 | AY740072 | 628/742 (84.6%) |
| *Moesziomyces aphidis ** | JCM10318 | JN942666 | 654/775 (84.4%) |
| *Moesziomyces aphidis ** | CBS517.83 | AF294699 | 654/775 (84.4%) |
| *Moesziomyces rugulosus* | CBS170.88 | JN942670 | 652/775 (84.1%) |
| *Ustilago striiformis* * | H.U.V.18286 | AY740172 | 466/486 (95.9%) |
| *Moesziomyces bullatus* | UstExs833M | AY740153 | 622/739 (84.2%) |
| *Anthracocystis apludae-aristatae* | MS287 | AY740045 | 618/732 (84.4%) |
| *Macalpinomyces spermophorus* * | F565 | AY740171 | 629/758 (83.0%) |
| *Sporisorium erythraeense* | Ust.Exs.849(M) | AY740049 | 622/744 (83.6%) |
| *Anthracocystis provincialis* | Ust.exs.759(M) | AY344988 | 620/741 (83.7%) |
| *Anthracocystis ovaria* | MP1871 | AY740020 | 620/743 (83.4%) |
| *Macalpinomyces tristachyae* | MS15 | AY740164 | 464/494 (93.9%) |
| *Ustilago shanxiensis* | AS2.2523 | DQ008956 | 445/463 (96.1%) |
| *Ustilago pamirica* | Ust.exs.789(M) | AY345005 | 464/494 (93.9%) |
| *Ustilago cynodontis* | MS199 | AY740168 | 460/488 (94.3%) |
| *Ustilago trichophora* | MS339 | AY740073 | 463/493 (93.9%) |
| *Ustilago bullata* | MP2363 | AY344998 | 463/494 (93.7%) |
| *Ustilago davisii* | HUV19252 | AY740169 | 459/493 (93.1%) |
| *Ustilago alcornii* | MS308 | AY740165 | 463/495 (93.5%) |
| *Langdonia jejuensis* | CBS10454 | EF079966 | 610/730 (83.6%) |
| *Macalpinomyces trichopterygis* | MS248 | AY740039 | 462/496 (93.1%) |
| *Melanopsichium pennsylvanicum* | HUV17548 | AY740040 | 463/499 (92.8%) |
| *Ustilago hordei* | Ust.exs.784 | AY345003 | 462/503 (91.8%) |
| *Ustilago calamagrostidis* | MS314 | AY740065 | 437/458 (95.4%) |
| *Ustilago xerochloae* | Ust.exs.1000(M) | AY345012 | 457/491 (93.1%) |
| *Ustilago sparsa* | KVU892 | JN367308 | 440/467 (94.2%) |

### [Table 13]

**Table 13**

| Registration name | Strain name | Accession No. | Homology rate |
|---|---|---|---|
| uncultured *Ustilaginales* | - | GU911148 | 698/733 (95.2%) |
| *Ustilago sporoboli-indici* | BRIP39706 | AY772736 | 673/698 (96.4%) |
| *Ustilago sporoboli-indici* * | BRIP65466 | MF716451 | 664/689 (96.4%) |
| *Ustilago sporoboli-indici* | - | MH474394 | 641/671 (95.5%) |
| *Ustilago shanxiensis* | CBS10075 | NR155995 | 696/767 (90.7%) |
| *Ustilago sporoboli-indici* * | CK768 | MH474458 | 606/619 (97.9%) |
| *Ustilago shanxiensis* | SN37 | FJ515182 | 690/765 (90.2%) |
| *Ustilago striiformis* | ISC 13766 | KF381024 | 691/768 (90.0%) |
| uncultured fungus | - | KF800238 | 692/774 (89.4%) |
| *Ustilago striiformis* | H.U.V. 18286 | AY740172 | 681/756 (90.1%) |
| uncultured fungus | - | KM877213 | 691/777 (88.9%) |
| *Ustilago sp.* | - | KX013166 | 690/771 (89.5%) |
| *Ustilago sp.* | CSR | KY284846 | 690/772 (89.4%) |
| *Ustilago sp.* | SMN03 | KF922222 | 689/773 (89.1%) |
| *Ustilago sp.* | SMN02 | KF922221 | 690/773 (89.3%) |
| uncultured *Ustilaginales* | - | GU910912 | 678/760 (89.2%) |
| *Ustilago striiformis* | - | KF381022 | 677/759 (89.2%) |
| *Ustilago cynodontis* | - | MH114998 | 681/764 (89.1%) |
| *Pseudozyma sp.* | MM 5 4 | KJ361485 | 683/766 (89.2%) |
| *Ustilago cynodontis* | - | KM213625 | 680/764 (89.0%) |
| *Ustilago nunavutica* | - | NR 132053 | 665/741 (89.7%) |
| *Ustilago shanxiensis ** | AS 2.2523 (ex-type) | DQ008956 | 667/742 (89.9%) |
| *Ustilago cynodontis* | - | KT988063 | 676/761 (88.8%) |
| *Ustilago striiformis* | - | KF381010 | 667/749 (89.1%) |
| *Ustilago sp.* | - | MH698491 | 669/751 (89.1%) |
| *Ustilago cynodontis* | - | MH114997 | 667/750 (88.9%) |
| *Ustilago cynodontis* | - | KC920744 | 667/750 (88.9%) |
| *Ustilago cynodontis* | - | KC920742 | 667/750 (88.9%) |
| *Ustilago striiformis* | - | KF381011 | 662/744 (89.0%) |
| *Ustilago cynodontis* | - | KT988064 | 668/752 (88.8%) |

Based on the above, EC431 strain was identified as Ustilago sp. closely related to Ustilago sporoboli-indici in the results of nucleotide sequence analysis for the 26S rDNA-D1/D2 region and the rDNA-ITS region.

The morphological properties of EC431 strain were examined through observation of the nature and morphology of the colonies. As a result of culturing on a YM plate medium for 4 days, cream to yellowish orange-colored colony nature with a smooth to wrinkled, butter-like, and wet surface was exhibited. It was confirmed that the nutritive cells were oval to cylindrical in shape, and that the proliferation was through budding from the short stalk of the cell pole part. No formation of sexual reproductive organs was observed in the plate after about 3 weeks from the start of culturing.

Based on the molecular phylogenetic position and morphological properties as well as the measurement of ergothioneine production, EC431 strain was determined to be a novel microorganism attributed to Ustilago sp. closely related to Ustilago sporoboli-indici.

### (Molecular phylogenetic position and morphological properties of EC171 strain)

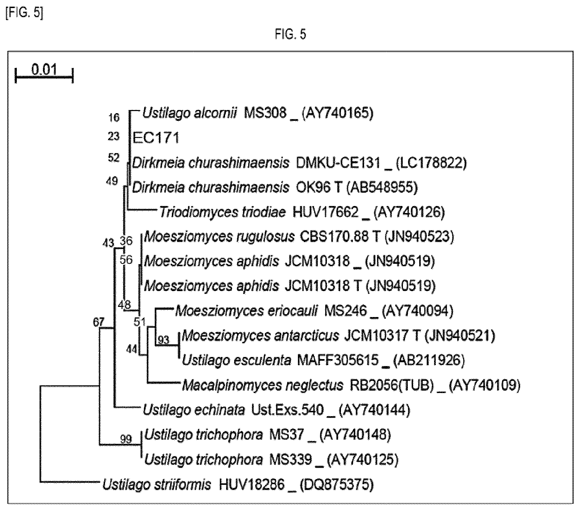

As a result of homology search by BLAST on DB-FU and International Nucleotide Sequence Databases using the microbial identification system "ENKI", the nucleotide sequence (SEQ ID NO: 5) of the 26S rDNA-D1/D2 region of EC171 strain showed 99.7 to 100% identity to a plurality of nucleotide sequences of Dirkmeia churashimaensis, which is a type of basidiomycete yeasts (Tables 14 and 15). In the molecular phylogenetic tree (FIG. 5) analyzed based on the nucleotide sequence obtained by homology search on DB-FU and International Nucleotide Sequence Databases, EC171 strain showed the same molecular phylogenetic position as that of a plurality of nucleotide sequences of Dirkmeia churashimaensis.

Table 14 shows the results of BLAST search on DB-FU, that is, the nucleotide sequence analysis data for the 26S rDNA-D1/D2 region found in the top 30 homology scores. The data with * is sequence data subjected to simplified molecular phylogenetic analysis.

Table 15 shows the results of a search by BLAST on International Nucleotide Sequence Databases, that is, the nucleotide sequence analysis data for the 26S rDNA-D1/D2 region found in the top 30 homology scores. The data with * is sequence data subjected to simplified molecular phylogenetic analysis. The strains with "^{a}" in Table 15 were excluded from the analysis because they are not with a nucleotide sequence derived from the reference strain, and the possibility of an error in the registration information was suggested.

FIG. 5 shows a simplified molecular phylogenetic tree based on the nucleotide sequence of the 26S rDNA-D1/D2 region of EC171 strain. The upper left line indicates the scale bar. The numbers positioned at the branches of the phylogenetic branches indicate bootstrap values. "T" at the end of a strain name indicates the type strain of the species.

### [Table 14]

**Table 14**

| Registration name | Strain name | Accession No. | Homology rate |
|---|---|---|---|
| *Dirkmeia churashimaensis ** | DMKU-CE131 | LC178822 | 605/605 (100.0%) |
| *Ustilago alcornii ** | MS308 | AY740165 | 603/605 (99.7%) |
| *Moesziomyces rugulosus ** | CBS170.88 | JN940523 | 603/605 (99.7%) |
| *Moesziomyces aphidis ** | JCM10318 | JN940519 | 603/605 (99.7%) |
| *Moesziomyces aphidis ** | JCM10318 | JN940519 | 603/605 (99.7%) |
| *Triodiomyces triodiae ** | HUV17662 | AY740126 | 602/605 (99.5%) |
| *Ustilago echinata ** | Ust.Exs.540 | AY740144 | 601/605 (99.3%) |
| *Moesziomyces eriocauli ** | MS246 | AY740094 | 599/605 (99.0%) |
| *Moesziomyces antarcticus ** | JCM10317 | JN940521 | 599/605 (99.0%) |
| *Macalpinomyces neglectus ** | RB2056(TUB) | AY740109 | 599/605 (99.0%) |
| *Ustilago esculenta ** | MAFF305615 | AB211926 | 598/604 (99.0%) |
| *Ustilago trichophora* * | MS37 | AY740148 | 598/605 (98.8%) |
| *Ustilago trichophora* * | MS339 | AY740125 | 598/605 (98.8%) |
| *Ustilago esculenta* | MS83 | AF453937 | 598/605 (98.8%) |
| *Ustilago affinis* | MP692 | AF133581 | 598/605 (98.8%) |
| *Moesziomyces bullatus* | CBS425.34 | DQ831011 | 598/605 (98.8%) |
| *Triodiomyces crassus* | CBS11336 | KY109978 | 599/605 (99.0%) |
| *Sporisorium sorghi* | MP2036a | AF009872 | 599/607 (98.7%) |
| *Sporisorium cruentum* | MS14 | AY740156 | 599/607 (98.7%) |
| *Ustilago trichophora* | MP1898 | AJ236141 | 597/605 (98.7%) |
| *Ustilago calamagrostidis* | 56518[M] | AY740119 | 597/605 (98.7%) |
| *Sporisorium andropogonis* | MS283 | AY740095 | 597/605 (98.7%) |
| *Anthracocystis elionuri* | MP2601 | AY740157 | 597/605 (98.7%) |
| *Anthracocystis provincialis* | Ust.exs.759(M) | AY747076 | 598/606 (98.7%) |
| *Sporisorium moniliferum* | MS98 | AF453940 | 597/605 (98.7%) |
| *Ustilago crameri* | MS72 | AY740143 | 592/598 (99.0%) |
| *Sporisorium foveolati* | MS21 | AY740103 | 598/607 (98.5%) |
| *Ustilago striiformis ** | HUV18286 | DQ875375 | 596/605 (98.5%) |
| *Sporisorium veracruzianum* | MP960 | AY740114 | 595/604 (98.5%) |
| *Sporisorium veracruzianum* | MP735 | AY740142 | 595/604 (98.5%) |

### [Table 15]

**Table 15**

| Registration name | Strain name | Accession No. | Homology rate |
|---|---|---|---|
| *Dirkmeia churashimaensis* | YE-162 | LC498467 | 605/605 (100.0%) |
| *Dirkmeia churashimaensis* | YE-157 | LC498464 | 605/605 (100.0%) |
| *Dirkmeia churashimaensis* | YE-149 | LC498460 | 605/605 (100.0%) |
| *Dirkmeia churashimaensis* | YE-121 | LC498442 | 605/605 (100.0%) |
| *Dirkmeia churashimaensis* | YE-36 | LC498414 | 605/605 (100.0%) |
| *Dirkmeia churashimaensis* | DMKU-RP81 | LC498270 | 605/605 (100.0%) |
| *Moesziomyces aphidis* ^{a} | - | MF062260 | 605/605 (100.0%) |
| *Dirkmeia churashimaensis* | DMKU-CE131 | LC178822 | 605/605 (100.0%) |
| *Dirkmeia churashimaensis* | DMKU-CE111 | LC178802 | 605/605 (100.0%) |
| *Dirkmeia churashimaensis* | DMKU-CE108 | LC178799 | 605/605 (100.0%) |
| *Dirkmeia churashimaensis* | DMKU-CE105 | LC178797 | 605/605 (100.0%) |
| *Dirkmeia churashimaensis* | - | LC178789 | 605/605 (100.0%) |
| *Dirkmeia churashimaensis* | DMKU-CE92 | LC178784 | 605/605 (100.0%) |
| *Dirkmeia churashimaensis* | DMKU-CE89 | LC178781 | 605/605 (100.0%) |
| *Dirkmeia churashimaensis* | DMKU-CE67 | LC178759 | 605/605 (100.0%) |
| *Dirkmeia churashimaensis* | - | LC178752 | 605/605 (100.0%) |
| *Dirkmeia churashimaensis* | DMKU-CE44 | LC178736 | 605/605 (100.0%) |
| *Dirkmeia churashimaensis* | DMKU-CE40 | LC178732 | 605/605 (100.0%) |
| *Dirkmeia churashimaensis* | DMKU-CE28 | LC178721 | 605/605 (100.0%) |
| *Dirkmeia churashimaensis* | DMKU-CE26 | LC178719 | 605/605 (100.0%) |
| *Dirkmeia churashimaensis* | DMKU-CE24 | LC178717 | 605/605 (100.0%) |
| *Dirkmeia churashimaensis* | - | LC178829 | 605/605 (100.0%) |
| *Pseudozyma sp.* | HB94-2 | KJ507299 | 605/605 (100.0%) |
| *fungal sp.* | N40 | MW131977 | 605/605 (100.0%) |
| *Moesziomyces aphidis* ^{a} | LN05 | AB617892 | 605/605 (100.0%) |
| *Dirkmeia churashimaensis* | DMKU-CP709 | LC430833 | 604/604 (100.0%) |
| *Moesziomyces aphidis* | - | AB772475 | 603/605 (99.7%) |
| *Moesziomyces aphidis* | CBS 517.83 | NG069796 | 603/605 (99.7%) |
| *Dirkmeia churashimaensis* | - | MT256146 | 603/605 (99.7%) |
| *Pseudozyma sp.* | - | MN533911 | 603/605 (99.7%) |

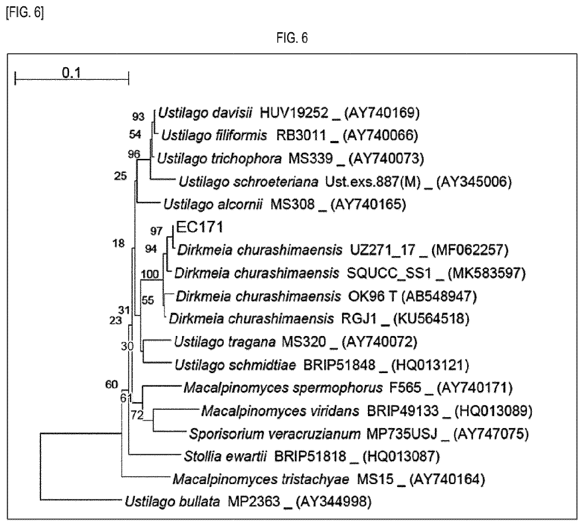

As a result of homology search by BLAST on DB-FU and International Nucleotide Sequence Databases using the microbial identification system "ENKI", the nucleotide sequence (SEQ ID NO: 6) of the rDNA-ITS region of EC171 strain showed 98.4 to 100% identity to a plurality of nucleotide sequences of Dirkmeia churashimaensis, which is a type of basidiomycete yeasts (Tables 16 and 17). In the molecular phylogenetic tree (FIG. 6) analyzed based on the nucleotide sequence obtained by homology search on DB-FU and International Nucleotide Sequence Databases, EC171 strain formed a cluster supported with a plurality of nucleotide sequences of Dirkmeia churashimaensis with a high bootstrap value of 100%.

Table 16 shows the results of a search by BLAST on DB-FU, that is, the nucleotide sequence analysis data for the rDNA-ITS region found in the top 30 homology scores. The data with * is sequence data subjected to simplified molecular phylogenetic analysis.

Table 17 shows the results of a search by BLAST on International Nucleotide Sequence Databases, that is, the nucleotide sequence analysis data for the rDNA-ITS region found in the top 30 homology scores. The data with * is sequence data subjected to simplified molecular phylogenetic analysis.

FIG. 6 shows a simplified molecular phylogenetic tree based on the nucleotide sequence of the rDNA-ITS region of EC171 strain. The upper left line indicates the scale bar. The numbers positioned at the branches of the phylogenetic branches indicate bootstrap values. "T" at the end of a strain name indicates the type strain of the species.

### [Table 16]

**Table 16**

| Registration name | Strain name | Accession No. | Homology rate |
|---|---|---|---|
| *Dirkmeia churashimaensis ** | OK96 | AB548947 | 692/703 (98.4%) |
| *Ustilago tragana ** | MS320 | AY740072 | 679/716 (94.8%) |
| *Ustilago davisii ** | HUV19252 | AY740169 | 683/728 (93.8%) |
| *Ustilago schmidtiae ** | BRIP51848 | HQ013121 | 689/749 (92.0%) |
| *Ustilago trichophora ** | MS339 | AY740073 | 673/717 (93.9%) |
| *Ustilago filiformis ** | RB3011 | AY740066 | 680/729 (93.3%) |
| *Ustilago alcornii ** | MS308 | AY740165 | 686/750 (91.5%) |
| *Macalpinomyces spermophorus ** | F565 | AY740171 | 668/734 (91.0%) |
| *Stollia ewartii* * | BRIP51818 | HQ013087 | 675/748 (90.2%) |
| *Macalpinomyces tristachyae ** | MS15 | AY740164 | 661/728 (90.8%) |
| *Ustilago schroeteriana** | Ust.exs.887(M) | AY345006 | 672/751 (89.5%) |
| *Macalpinomyces viridans* * | BRIP49133 | HQ013089 | 663/740 (89.6%) |
| *Sporisorium veracruzianum** | MP735USJ | AY747075 | 639/699 (91.4%) |
| *Macalpinomyces loudetiae* | MS250 | AY740151 | 650/721 (90.2%) |
| *Ustilago bullata ** | MP2363 | AY344998 | 674/768 (87.8%) |
| *Ustilago striiformis* | H.U.V.18286 | AY740172 | 667/754 (88.5%) |
| *Pseudozyma hubeiensis* | CBS10077 | DQ008954 | 637/705 (90.4%) |
| *Pseudozyma alboarmeniaca* | DMST17135 | AB117961 | 644/708 (91.0%) |
| *Ustilago austro-africana* | MS316 | AY740061 | 659/747 (88.2%) |
| *Ustilago pamirica* | Ust.exs.789(M) | AY345005 | 661/751 (88.0%) |
| *Stollia bursa* | KVU844 | JN367291 | **614/668** (91.9%) |
| *Macalpinomyces trichopterygis* | MS248 | AY740039 | 629/703 (89.5%) |
| *Ustilago esculenta* | MAFF305615 | AB211926 | 676/774 (87.3%) |
| *Sporisorium veracruzianum* | MP960 | AY344993 | 611/669 (91.3%) |
| *Moesziomyces antarcticus* | JCM10317 | JN942668 | 676/774 (87.3%) |
| *Ustilago bouriqueti* | MS315 | AY740167 | 662/743 (89.1%) |
| *Ustilago vetiveriae* | H.U.V.17954 | AY345011 | 632/712 (88.8%) |
| *Sporisorium trachypogonis-plumosi* | MS281 | AY740060 | 634/700 (90.6%) |
| *Ustilago esculenta* | MS83 | AY345002 | 548/577 (95.0%) |
| *Ustilago hordei* | Ust.exs.784 | AY345003 | 660/763 (86.5%) |

### [Table 17]

**Table 17**

| Registration name | Strain name | Accession No. | Homology rate |
|---|---|---|---|
| *Dirkmeia sp.* | Y75 | MN515015 | 732/732 (100.0%) |
| *Dirkmeia churashimaensis* | Y78 | MN515013 | 732/732 (100.0%) |
| uncultured fungus | - | KX515770 | 732/732 (100.0%) |
| uncultured fungus | - | KX515470 | 732/732 (100.0%) |
| uncultured fungus | - | KX515432 | 732/732 (100.0%) |
| *fungal sp.* | N40 | MW131977 | 732/732 (100.0%) |
| *Dirkmeia sp.* | Y6 | MN515021 | 731/732 (99.9%) |
| *Dirkmeia sp.* | Y70 | MN515018 | 731/732 (99.9%) |
| *Dirkmeia sp.* | - | MN515012 | 731/732 (99.9%) |
| *Dirkmeia churashimaensis ** | UZ271 17 | MF062257 | 731/732 (99.9%) |
| *Dirkmeia sp.* | M19 | MK651592 | 723/723 (100.0%) |
| uncultured fungus | - | KX515635 | 728/731 (99.6%) |
| *Dirkmeia sp.* | - | MK294224 | 728/732 (99.5%) |
| *fungal sp.* | SV657 | KP757563 | 718/719 (99.9%) |
| *Dirkmeia sp.* | Y9 | MN515020 | 727/736 (98.8%) |
| uncultured fungus | - | KX515629 | 725/736 (98.5%) |
| uncultured fungus | - | KX515626 | 724/736 (98.4%) |
| uncultured fungus | - | KX515578 | 724/736 (98.4%) |
| uncultured fungus | - | KX515487 | 724/736 (98.4%) |
| *Dirkmeia churashimaensis ** | RGJ1 | KU564518 | 688/688 (100.0%) |
| *Dirkmeia churashimaensis ** | SQUCC SS1 | MK583597 | **682/684** (99.7%) |
| *Dirkmeia churashimaensis* | - | MN758679 | 678/680 (99.7%) |
| *Dirkmeia churashimaensis* | - | MN758678 | 678/680 (99.7%) |
| *Dirkmeia churashimaensis* | - | MN758677 | 678/680 (99.7%) |
| *Dirkmeia churashimaensis* | - | MN758676 | 678/680 (99.7%) |
| *Dirkmeia churashimaensis* | - | MN758675 | 678/680 (99.7%) |
| *Dirkmeia churashimaensis* | - | MN758674 | 678/680 (99.7%) |
| *Dirkmeia churashimaensis* | - | MN758673 | 678/680 (99.7%) |
| *Dirkmeia churashimaensis* | - | MN758672 | 678/680 (99.7%) |
| *Dirkmeia churashimaensis* | - | MN758671 | 678/680 (99.7%) |

Based on the above, EC171 strain was identified as Dirkmeia churashimaensis in the results of nucleotide sequence analysis for the 26S rDNA-D1/D2 region and the rDNA-ITS region.

The morphological properties of EC171 strain were examined through observation of the nature and morphology of the colonies. As a result of culturing on a YM plate medium for 4 days, yellowish orange to cream-colored colony nature with a smooth, butter-like, wet, and viscous surface was exhibited. It was confirmed that the nutritive cells were oval to ovoid in shape, and that the proliferation was through budding from the short stalk of the cell pole part. No formation of sexual reproductive organs was observed in the plate after about 3 weeks from the start of culturing.

Based on the molecular phylogenetic position and morphological properties as well as the measurement of ergothioneine production, EC171 strain was determined to be a novel microorganism attributed to Dirkmeia churashimaensis.

### (Molecular phylogenetic position and morphological properties of EC581 strain)

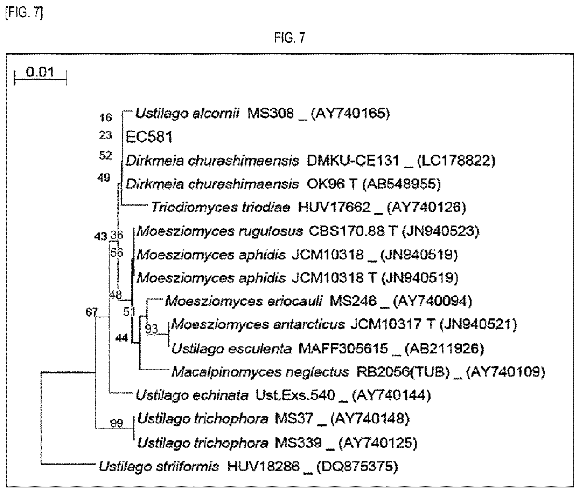

As a result of homology search by BLAST on DB-FU and International Nucleotide Sequence Databases using the microbial identification system "ENKI", the nucleotide sequence (SEQ ID NO: 7) of the 26S rDNA-D1/D2 region of EC581 strain showed 99.7 to 100% identity to a plurality of nucleotide sequences of Dirkmeia churashimaensis, which is a type of basidiomycete yeasts (Tables 18 and 19). In the molecular phylogenetic tree (FIG. 7) analyzed based on the nucleotide sequence obtained by homology search on DB-FU and International Nucleotide Sequence Databases, EC581 strain showed the same molecular phylogenetic position as that of a plurality of nucleotide sequences of Dirkmeia churashimaensis.

Table 18 shows the results of a search by BLAST on DB-FU, that is, the nucleotide sequence analysis data for the 26S rDNA-D1/D2 region found in the top 30 homology scores. The data with * is sequence data subjected to simplified molecular phylogenetic analysis.

Table 19 shows the results of a search by BLAST on International Nucleotide Sequence Databases, that is, the nucleotide sequence analysis data for the 26S rDNA-D1/D2 region found in the top 30 homology scores. The data with * is sequence data subjected to simplified molecular phylogenetic analysis. The strains with "^{a}" in Table 19 were excluded from the analysis because they are not with a nucleotide sequence derived from the reference strain, and the possibility of an error in the registration information was suggested.

FIG. 7 shows a simplified molecular phylogenetic tree based on the nucleotide sequence of the 26S rDNA-D1/D2 region of EC581 strain. The upper left line indicates the scale bar. The numbers positioned at the branches of the phylogenetic branches indicate bootstrap values. "T" at the end of a strain name indicates the type strain of the species.

### [Table 18]

**Table 18**

| Registration name | Strain name | Accession No. | Homology rate |
|---|---|---|---|
| *Dirkmeia churashimaensis ** | DMKU-CE131 | LC178822 | 605/605 (100.0%) |
| *Ustilago alcornii ** | MS308 | AY740165 | 603/605 (99.7%) |
| *Moesziomyces rugulosus ** | CBS170.88 | JN940523 | 603/605 (99.7%) |
| *Moesziomyces aphidis ** | JCM10318 | JN940519 | 603/605 (99.7%) |
| *Moesziomyces aphidis ** | JCM10318 | JN940519 | 603/605 (99.7%) |
| *Triodiomyces triodiae ** | HUV17662 | AY740126 | 602/605 (99.5%) |
| *Ustilago echinata ** | Ust.Exs.540 | AY740144 | 601/605 (99.3%) |
| *Moesziomyces eriocauli ** | MS246 | AY740094 | 599/605 (99.0%) |
| *Moesziomyces antarcticus ** | JCM10317 | JN940521 | 599/605 (99.0%) |
| *Macalpinomyces neglectus ** | RB2056(TUB) | AY740109 | 599/605 (99.0%) |
| *Ustilago esculenta ** | MAFF305615 | AB211926 | 598/604 (99.0%) |
| *Ustilago trichophora* * | MS37 | AY740148 | 598/605 (98.8%) |
| *Ustilago trichophora* * | MS339 | AY740125 | 598/605 (98.8%) |
| *Ustilago esculenta* | MS83 | AF453937 | 598/605 (98.8%) |
| *Ustilago affinis* | MP692 | AF133581 | 598/605 (98.8%) |
| *Moesziomyces bullatus* | CBS425.34 | DQ831011 | 598/605 (98.8%) |
| *Triodiomyces crassus* | CBS11336 | KY109978 | 599/605 (99.0%) |
| *Sporisorium sorghi* | MP2036a | AF009872 | 599/607 (98.7%) |
| *Sporisorium cruentum* | MS14 | AY740156 | 599/607 (98.7%) |
| *Ustilago trichophora* | MP1898 | AJ236141 | 597/605 (98.7%) |
| *Ustilago calamagrostidis* | 56518[M] | AY740119 | 597/605 (98.7%) |
| *Sporisorium andropogonis* | MS283 | AY740095 | 597/605 (98.7%) |
| *Anthracocystis elionuri* | MP2601 | AY740157 | 597/605 (98.7%) |
| *Anthracocystis provincialis* | Ust.exs.759(M) | AY747076 | 598/606 (98.7%) |
| *Sporisorium moniliferum* | MS98 | AF453940 | 597/605 (98.7%) |
| *Ustilago crameri* | MS72 | AY740143 | 592/598 (99.0%) |
| *Sporisorium foveolati* | MS21 | AY740103 | 598/607 (98.5%) |
| *Ustilago striiformis ** | HUV18286 | DQ875375 | 596/605 (98.5%) |
| *Sporisorium veracruzianum* | MP960 | AY740114 | 595/604 (98.5%) |
| *Sporisorium veracruzianum* | MP735 | AY740142 | 595/604 (98.5%) |

### [Table 19]

**Table 19**

| Registration name | Strain name | Accession No. | Homology rate |
|---|---|---|---|
| *Dirkmeia churashimaensis* | YE-162 | LC498467 | 605/605 (100.0%) |
| *Dirkmeia churashimaensis* | YE-157 | LC498464 | 605/605 (100.0%) |
| *Dirkmeia churashimaensis* | YE-149 | LC498460 | 605/605 (100.0%) |
| *Dirkmeia churashimaensis* | YE-121 | LC498442 | 605/605 (100.0%) |
| *Dirkmeia churashimaensis* | YE-36 | LC498414 | 605/605 (100.0%) |
| *Dirkmeia churashimaensis* | DMKU-RP81 | LC498270 | 605/605 (100.0%) |
| *Moesziomyces aphidis* ^{a} | - | MF062260 | 605/605 (100.0%) |
| *Dirkmeia churashimaensis* | DMKU-CE131 | LC178822 | 605/605 (100.0%) |
| *Dirkmeia churashimaensis* | DMKU-CE111 | LC178802 | 605/605 (100.0%) |
| *Dirkmeia churashimaensis* | - | LC178799 | 605/605 (100.0%) |
| *Dirkmeia churashimaensis* | DMKU-CE105 | LC178797 | 605/605 (100.0%) |
| *Dirkmeia churashimaensis* | DMKU-CE97 | LC178789 | 605/605 (100.0%) |
| *Dirkmeia churashimaensis* | DMKU-CE92 | LC178784 | 605/605 (100.0%) |
| *Dirkmeia churashimaensis* | DMKU-CE89 | LC178781 | 605/605 (100.0%) |
| *Dirkmeia churashimaensis* | DMKU-CE67 | LC178759 | 605/605 (100.0%) |
| *Dirkmeia churashimaensis* | DMKU-CE60 | LC178752 | 605/605 (100.0%) |
| *Dirkmeia churashimaensis* | DMKU-CE44 | LC178736 | 605/605 (100.0%) |
| *Dirkmeia churashimaensis* | DMKU-CE40 | LC178732 | 605/605 (100.0%) |
| *Dirkmeia churashimaensis* | DMKU-CE28 | LC178721 | 605/605 (100.0%) |
| *Dirkmeia churashimaensis* | DMKU-CE26 | LC178719 | 605/605 (100.0%) |
| *Dirkmeia churashimaensis* | - | LC178717 | 605/605 (100.0%) |
| *Dirkmeia churashimaensis* | DMKU-CE138 | LC178829 | 605/605 (100.0%) |
| *Pseudozyma sp.* | HB94-2 | KJ507299 | 605/605 (100.0%) |
| *fungal sp.* | N40 | MW131977 | 605/605 (100.0%) |
| *Moesziomyces aphidis* ^{a} | LN05 | AB617892 | 605/605 (100.0%) |
| *Dirkmeia churashimaensis* | DMKU-CP709 | LC430833 | 604/604 (100.0%) |
| *Moesziomyces aphidis* | DMKU-RK86 | AB772475 | 603/605 (99.7%) |
| *Moesziomyces aphidis* | CBS 517.83 | NG069796 | 603/605 (99.7%) |
| *Dirkmeia churashimaensis* | - | MT256146 | 603/605 (99.7%) |
| *Pseudozyma sp.* | - | MN533911 | 603/605 (99.7%) |

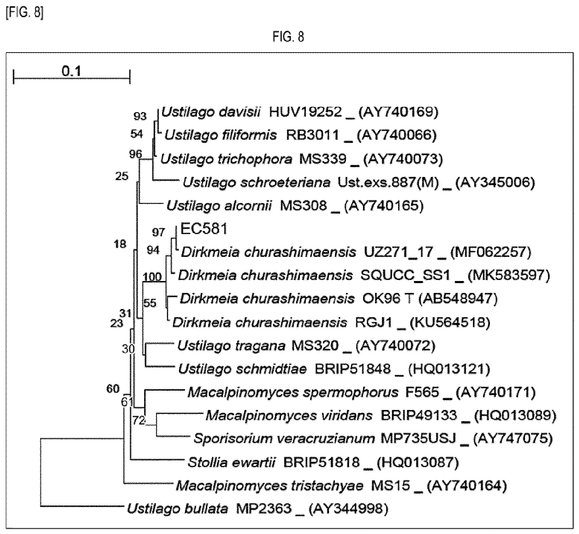

As a result of homology search by BLAST on DB-FU and International Nucleotide Sequence Databases using the microbial identification system "ENKI", the nucleotide sequence (SEQ ID NO: 8) of the rDNA-ITS region of EC581 strain showed 98.4 to 100% identity to a plurality of nucleotide sequences of Dirkmeia churashimaensis, which is a type of basidiomycete yeasts (Tables 20 and 21). In the molecular phylogenetic tree (FIG. 8) analyzed based on the nucleotide sequence obtained by homology search on DB-FU and International Nucleotide Sequence Databases, EC581 strain formed a cluster supported with a plurality of nucleotide sequences of Dirkmeia churashimaensis with a high bootstrap value of 100%.

Table 20 shows the results of a search by BLAST on DB-FU, that is, the nucleotide sequence analysis data for the rDNA-ITS region found in the top 30 homology scores. The data with * is sequence data subjected to simplified molecular phylogenetic analysis.

Table 21 shows the results of a search by BLAST on International Nucleotide Sequence Databases, that is, the nucleotide sequence analysis data for the rDNA-ITS region found in the top 30 homology scores. The data with * is sequence data subjected to simplified molecular phylogenetic analysis.

FIG. 8 shows a simplified molecular phylogenetic tree based on the nucleotide sequence of the rDNA-ITS region of EC581 strain. The upper left line indicates the scale bar. The numbers positioned at the branches of the phylogenetic branches indicate bootstrap values. "T" at the end of a strain name indicates the type strain of the species.

### [Table 20]

**Table 20**

| Registration name | Strain name | Accession No. | Homology rate |
|---|---|---|---|
| *Dirkmeia churashimaensis ** | OK96 | AB548947 | 692/703 (98.4%) |
| *Ustilago tragana ** | MS320 | AY740072 | 679/716 (94.8%) |
| *Ustilago davisii ** | HUV19252 | AY740169 | 683/728 (93.8%) |
| *Ustilago schmidtiae* * | BRIP51848 | HQ013121 | 689/749 (92.0%) |
| *Ustilago trichophora* * | MS339 | AY740073 | 673/717 (93.9%) |
| *Ustilago filiformis* * | RB3011 | AY740066 | 680/729 (93.3%) |
| *Ustilago alcornii ** | MS308 | AY740165 | 686/750 (91.5%) |
| *Macalpinomyces spermophorus* * | F565 | AY740171 | 668/734 (91.0%) |
| *Stollia ewartii ** | BRIP51818 | HQ013087 | 675/748 (90.2%) |
| *Macalpinomyces tristachyae ** | MS15 | AY740164 | 661/728 (90.8%) |
| *Ustilago schroeteriana* * | Ust.exs.887(M) | AY345006 | 672/751 (89.5%) |
| *Macalpinomyces viridans* * | BRIP49133 | HQ013089 | 663/740 (89.6%) |
| *Sporisorium veracruzianum** | MP735USJ | AY747075 | 639/699 (91.4%) |
| *Macalpinomyces loudetiae* | MS250 | AY740151 | 650/721 (90.2%) |
| *Ustilago bullata ** | MP2363 | AY344998 | 674/768 (87.8%) |
| *Ustilago striiformis* | H.U.V.18286 | AY740172 | 667/754 (88.5%) |
| *Pseudozyma hubeiensis* | CBS10077 | DQ008954 | 637/705 (90.4%) |
| *Pseudozyma alboarmeniaca* | DMST17135 | AB117961 | 644/708 (91.0%) |
| *Ustilago austro-africana* | MS316 | AY740061 | 659/747 (88.2%) |
| *Ustilago pamirica* | Ust.exs.789(M) | AY345005 | 661/751 (88.0%) |
| *Stollia bursa* | KVU844 | JN367291 | **614/668** (91.9%) |
| *Macalpinomyces trichopterygis* | MS248 | AY740039 | 629/703 (89.5%) |
| *Ustilago esculenta* | MAFF305615 | AB211926 | 676/774 (87.3%) |
| *Sporisorium veracruzianum* | MP960 | AY344993 | 611/669 (91.3%) |
| *Moesziomyces antarcticus* | JCM10317 | JN942668 | 676/774 (87.3%) |
| *Ustilago bouriqueti* | MS315 | AY740167 | 662/743 (89.1%) |
| *Ustilago vetiveriae* | H.U.V.17954 | AY345011 | 632/712 (88.8%) |
| *Sporisorium trachypogonis-plumosi* | MS281 | AY740060 | 634/700 (90.6%) |
| *Ustilago esculenta* | MS83 | AY345002 | 548/577 (95.0%) |
| *Ustilago hordei* | Ust.exs.784 | AY345003 | 660/763 (86.5%) |

### [Table 21]

**Table 21**

| Registration name | Strain name | Accession No. | Homology rate |
|---|---|---|---|
| *Dirkmeia sp.* | Y75 | MN515015 | 732/732 (100.0%) |
| *Dirkmeia churashimaensis* | Y78 | MN515013 | 732/732 (100.0%) |
| uncultured fungus | - | KX515770 | 732/732 (100.0%) |
| uncultured fungus | - | KX515470 | 732/732 (100.0%) |
| uncultured fungus | - | KX515432 | 732/732 (100.0%) |
| *fungal sp.* | N40 | MW131977 | 732/732 (100.0%) |
| *Dirkmeia sp.* | Y6 | MN515021 | 731/732 (99.9%) |
| *Dirkmeia sp.* | Y70 | MN515018 | 731/732 (99.9%) |
| *Dirkmeia sp.* | Y79 | MN515012 | 731/732 (99.9%) |
| *Dirkmeia churashimaensis ** | UZ271 17 | MF062257 | 731/732 (99.9%) |
| *Dirkmeia sp.* | - | MK651592 | 723/723 (100.0%) |
| uncultured fungus | - | KX515635 | 728/731 (99.6%) |
| *Dirkmeia sp.* | - | MK294224 | 728/732 (99.5%) |
| *fungal sp.* | SV657 | KP757563 | 718/719 (99.9%) |
| *Dirkmeia sp.* | Y9 | MN515020 | 727/736 (98.8%) |
| uncultured fungus | - | KX515629 | 725/736 (98.5%) |
| uncultured fungus | - | KX515626 | 724/736 (98.4%) |
| uncultured fungus | - | KX515578 | 724/736 (98.4%) |
| uncultured fungus | - | KX515487 | 724/736 (98.4%) |
| *Dirkmeia churashimaensis ** | RGJ1 | KU564518 | 688/688 (100.0%) |
| *Dirkmeia churashimaensis ** | SQUCC SS1 | MK583597 | 682/684 (99.7%) |
| *Dirkmeia churashimaensis* | - | MN758679 | 678/680 (99.7%) |
| *Dirkmeia churashimaensis* | - | MN758678 | 678/680 (99.7%) |
| *Dirkmeia churashimaensis* | - | MN758677 | 678/680 (99.7%) |
| *Dirkmeia churashimaensis* | - | MN758676 | 678/680 (99.7%) |
| *Dirkmeia churashimaensis* | - | MN758675 | 678/680 (99.7%) |
| *Dirkmeia churashimaensis* | - | MN758674 | 678/680 (99.7%) |
| *Dirkmeia churashimaensis* | - | MN758673 | 678/680 (99.7%) |
| *Dirkmeia churashimaensis* | - | MN758672 | 678/680 (99.7%) |
| *Dirkmeia churashimaensis* | - | MN758671 | 678/680 (99.7%) |

Based on the above, EC581 strain was identified as Dirkmeia churashimaensis in the results of nucleotide sequence analysis for the 26S rDNA-D1/D2 region and the rDNA-ITS region.

The morphological properties of EC581 strain were examined through observation of the nature and morphology of the colonies. As a result of culturing on a YM plate medium for 4 days, yellowish orange to cream-colored colony nature with a smooth, butter-like, wet, and viscous surface was exhibited. It was confirmed that the nutritive cells were oval to ovoid in shape, and that the proliferation was through budding from the short stalk of the cell local part. No formation of sexual reproductive organs was observed in the plate after about 3 weeks from the start of culturing.

Based on the molecular phylogenetic position and morphological properties as well as the measurement of ergothioneine production, EC581 strain was determined to be a novel microorganism attributed to Dirkmeia churashimaensis.

### (Molecular phylogenetic position and morphological properties of EC592 strain)

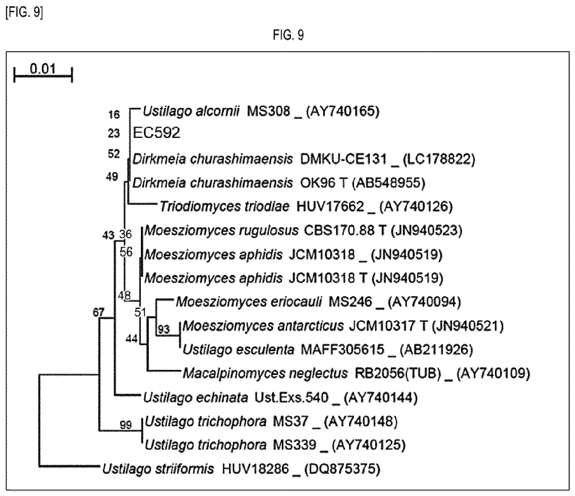

As a result of homology search by BLAST on DB-FU and International Nucleotide Sequence Databases using the microbial identification system "ENKI", the nucleotide sequence (SEQ ID NO: 9) of the 26S rDNA-D1/D2 region of EC592 strain showed 99.7 to 100% identity to a plurality of nucleotide sequences of Dirkmeia churashimaensis, which is a type of basidiomycete yeasts (Tables 22 and 23). In the molecular phylogenetic tree (FIG. 9) analyzed based on the nucleotide sequence obtained by homology search on DB-FU and International Nucleotide Sequence Databases, EC592 strain showed the same molecular phylogenetic position as that of a plurality of nucleotide sequences of Dirkmeia churashimaensis.

Table 22 shows the results of a search by BLAST on DB-FU, that is, the nucleotide sequence analysis data for the 26S rDNA-D1/D2 region found in the top 30 homology scores. The data with * is sequence data subjected to simplified molecular phylogenetic analysis.

Table 23 shows the results of a search by BLAST on International Nucleotide Sequence Databases, that is, the nucleotide sequence analysis data for the 26S rDNA-D1/D2 region found in the top 30 homology scores. The data with * is sequence data subjected to simplified molecular phylogenetic analysis. The strains with "^{a}" in Table 23 were excluded from the analysis because they are not with a nucleotide sequence derived from the reference strain, and the possibility of an error in the registration information was suggested.

FIG. 9 shows a simplified molecular phylogenetic tree based on the nucleotide sequence of the 26S rDNA-D1/D2 region of EC592 strain. The upper left line indicates the scale bar. The numbers positioned at the branches of the phylogenetic branches indicate bootstrap values. "T" at the end of a strain name indicates the type strain of the species.

### [Table 22]

**Table 22**

| Registration name | Strain name | Accession No. | Homology rate |
|---|---|---|---|
| *Dirkmeia churashimaensis ** | DMKU-CE131 | LC178822 | 605/605 (100.0%) |
| *Ustilago alcornii ** | MS308 | AY740165 | 603/605 (99.7%) |
| *Moesziomyces rugulosus ** | CBS170.88 | JN940523 | 603/605 (99.7%) |
| *Moesziomyces aphidis ** | JCM10318 | JN940519 | 603/605 (99.7%) |
| *Moesziomyces aphidis ** | JCM10318 | JN940519 | 603/605 (99.7%) |
| *Triodiomyces triodiae ** | HUV17662 | AY740126 | 602/605 (99.5%) |
| *Ustilago echinata ** | Ust.Exs.540 | AY740144 | 601/605 (99.3%) |
| *Moesziomyces eriocauli ** | MS246 | AY740094 | 599/605 (99.0%) |
| *Moesziomyces antarcticus ** | JCM10317 | JN940521 | 599/605 (99.0%) |
| *Macalpinomyces neglectus ** | RB2056(TUB) | AY740109 | 599/605 (99.0%) |
| *Ustilago esculenta ** | MAFF305615 | AB211926 | 598/604 (99.0%) |
| *Ustilago trichophora* * | MS37 | AY740148 | 598/605 (98.8%) |
| *Ustilago trichophora* * | MS339 | AY740125 | 598/605 (98.8%) |
| *Ustilago esculenta* | MS83 | AF453937 | 598/605 (98.8%) |
| *Ustilago affinis* | MP692 | AF133581 | 598/605 (98.8%) |
| *Moesziomyces bullatus* | CBS425.34 | DQ831011 | 598/605 (98.8%) |
| *Triodiomyces crassus* | CBS11336 | KY109978 | 599/605 (99.0%) |
| *Sporisorium sorghi* | MP2036a | AF009872 | 599/607 (98.7%) |
| *Sporisorium cruentum* | MS14 | AY740156 | 599/607 (98.7%) |
| *Ustilago trichophora* | MP1898 | AJ236141 | 597/605 (98.7%) |
| *Ustilago calamagrostidis* | 56518[M] | AY740119 | 597/605 (98.7%) |
| *Sporisorium andropogonis* | MS283 | AY740095 | 597/605 (98.7%) |
| *Anthracocystis elionuri* | MP2601 | AY740157 | 597/605 (98.7%) |
| *Anthracocystis provincialis* | Ust.exs.759(M) | AY747076 | 598/606 (98.7%) |
| *Sporisorium moniliferum* | MS98 | AF453940 | 597/605 (98.7%) |
| *Ustilago crameri* | MS72 | AY740143 | 592/598 (99.0%) |
| *Sporisorium foveolati* | MS21 | AY740103 | 598/607 (98.5%) |
| *Ustilago striiformis ** | HUV18286 | DQ875375 | 596/605 (98.5%) |
| *Sporisorium veracruzianum* | MP960 | AY740114 | 595/604 (98.5%) |
| *Sporisorium veracruzianum* | MP735 | AY740142 | 595/604 (98.5%) |

### [Table 23]

**Table 23**

| Registration name | Strain name | Accession No. | Homology rate |
|---|---|---|---|
| *Dirkmeia churashimaensis* | YE-162 | LC498467 | 605/605 (100.0%) |
| *Dirkmeia churashimaensis* | YE-157 | LC498464 | 605/605 (100.0%) |
| *Dirkmeia churashimaensis* | YE-149 | LC498460 | 605/605 (100.0%) |
| *Dirkmeia churashimaensis* | YE-121 | LC498442 | 605/605 (100.0%) |
| *Dirkmeia churashimaensis* | YE-36 | LC498414 | 605/605 (100.0%) |
| *Dirkmeia churashimaensis* | DMKU-RP81 | LC498270 | 605/605 (100.0%) |
| *Moesziomyces aphidis* ^{a} | - | MF062260 | 605/605 (100.0%) |
| *Dirkmeia churashimaensis* | DMKU-CE131 | LC178822 | 605/605 (100.0%) |
| *Dirkmeia churashimaensis* | DMKU-CE111 | LC178802 | 605/605 (100.0%) |
| *Dirkmeia churashimaensis* | DMKU-CE108 | LC178799 | 605/605 (100.0%) |
| *Dirkmeia churashimaensis* | DMKU-CE105 | LC178797 | 605/605 (100.0%) |
| *Dirkmeia churashimaensis* | DMKU-CE97 | LC178789 | 605/605 (100.0%) |
| *Dirkmeia churashimaensis* | DMKU-CE92 | LC178784 | 605/605 (100.0%) |
| *Dirkmeia churashimaensis* | DMKU-CE89 | LC178781 | 605/605 (100.0%) |
| *Dirkmeia churashimaensis* | DMKU-CE67 | LC178759 | 605/605 (100.0%) |
| *Dirkmeia churashimaensis* | DMKU-CE60 | LC178752 | 605/605 (100.0%) |
| *Dirkmeia churashimaensis* | DMKU-CE44 | LC178736 | 605/605 (100.0%) |
| *Dirkmeia churashimaensis* | DMKU-CE40 | LC178732 | 605/605 (100.0%) |
| *Dirkmeia churashimaensis* | DMKU-CE28 | LC178721 | 605/605 (100.0%) |
| *Dirkmeia churashimaensis* | DMKU-CE26 | LC178719 | 605/605 (100.0%) |
| *Dirkmeia churashimaensis* | DMKU-CE24 | LC178717 | 605/605 (100.0%) |
| *Dirkmeia churashimaensis* | DMKU-CE138 | LC178829 | 605/605 (100.0%) |
| *Pseudozyma sp.* | HB94-2 | KJ507299 | 605/605 (100.0%) |
| *fungal sp.* | N40 | MW131977 | 605/605 (100.0%) |
| *Moesziomyces aphidis* ^{a} | LN05 | AB617892 | 605/605 (100.0%) |
| *Dirkmeia churashimaensis* | DMKU-CP709 | LC430833 | 604/604 (100.0%) |
| *Moesziomyces aphidis* | DMKU-RK86 | AB772475 | 603/605 (99.7%) |
| *Moesziomyces aphidis* | CBS 517.83 | NG069796 | 603/605 (99.7%) |
| *Dirkmeia churashimaensis* | - | MT256146 | 603/605 (99.7%) |
| *Pseudozyma sp.* | - | MN533911 | 603/605 (99.7%) |

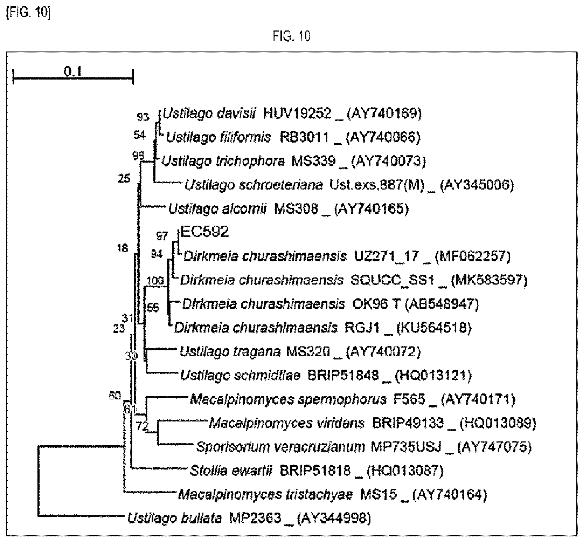

As a result of homology search by BLAST on DB-FU and International Nucleotide Sequence Databases using the microbial identification system "ENKI", the nucleotide sequence (SEQ ID NO: 10) of the rDNA-ITS region of EC592 strain showed 98.4 to 100% identity to a plurality of nucleotide sequences of Dirkmeia churashimaensis, which is a type of basidiomycete yeasts (Tables 24 and 25). In the molecular phylogenetic tree (FIG. 10) analyzed based on the nucleotide sequence obtained by homology search on DB-FU and International Nucleotide Sequence Databases, EC592 strain formed a cluster supported with a plurality of nucleotide sequences of Dirkmeia churashimaensis with a high bootstrap value of 100%.

Table 24 shows the results of a search by BLAST on DB-FU, that is, the nucleotide sequence analysis data for the rDNA-ITS region found in the top 30 homology scores. The data with * is sequence data subjected to simplified molecular phylogenetic analysis.

Table 25 shows the results of a search by BLAST on International Nucleotide Sequence Databases, that is, the nucleotide sequence analysis data for the rDNA-ITS region found in the top 30 homology scores. The data with * is sequence data subjected to simplified molecular phylogenetic analysis.

FIG. 10 shows a simplified molecular phylogenetic tree based on the nucleotide sequence of the rDNA-ITS region of EC592 strain. The upper left line indicates the scale bar. The numbers positioned at the branches of the phylogenetic branches indicate bootstrap values. "T" at the end of a strain name indicates the type strain of the species.

### [Table 24]

**Table 24**

| Registration name | Strain name | Accession No. | Homology rate |
|---|---|---|---|
| *Dirkmeia churashimaensis ** | OK96 | AB548947 | 692/703 (98.4%) |
| *Ustilago tragana ** | MS320 | AY740072 | 679/716 (94.8%) |
| *Ustilago davisii ** | HUV19252 | AY740169 | 683/728 (93.8%) |
| *Ustilago schmidtiae* * | BRIP51848 | HQ013121 | 689/749 (92.0%) |
| *Ustilago trichophora* * | MS339 | AY740073 | 673/717 (93.9%) |
| *Ustilago filiformis* * | RB3011 | AY740066 | 680/729 (93.3%) |
| *Ustilago alcornii ** | MS308 | AY740165 | 686/750 (91.5%) |
| *Macalpinomyces spermophorus* * | F565 | AY740171 | 668/734 (91.0%) |
| *Stollia ewartii ** | BRIP51818 | HQ013087 | 675/748 (90.2%) |
| *Macalpinomyces tristachyae ** | MS15 | AY740164 | 661/728 (90.8%) |
| *Ustilago schroeteriana** | Ust.exs.887(M) | AY345006 | 672/751 (89.5%) |
| *Macalpinomyces viridans ** | BRIP49133 | HQ013089 | 663/740 (89.6%) |
| *Sporisorium veracruzianum** | MP735USJ | AY747075 | 639/699 (91.4%) |
| *Macalpinomyces loudetiae* | MS250 | AY740151 | 650/721 (90.2%) |
| *Ustilago bullata ** | MP2363 | AY344998 | 674/768 (87.8%) |
| *Ustilago striiformis* | H.U.V.18286 | AY740172 | 667/754 (88.5%) |
| *Pseudozyma hubeiensis* | CBS10077 | DQ008954 | 637/705 (90.4%) |
| *Pseudozyma alboarmeniaca* | DMST17135 | AB117961 | 644/708 (91.0%) |
| *Ustilago austro-africana* | MS316 | AY740061 | 659/747 (88.2%) |
| *Ustilago pamirica* | Ust.exs.789(M) | AY345005 | 661/751 (88.0%) |
| *Stollia bursa* | KVU844 | JN367291 | 614/668 (91.9%) |
| *Macalpinomyces trichopterygis* | MS248 | AY740039 | 629/703 (89.5%) |
| *Ustilago esculenta* | MAFF305615 | AB211926 | 676/774 (87.3%) |
| *Sporisorium veracruzianum* | MP960 | AY344993 | 611/669 (91.3%) |
| *Moesziomyces antarcticus* | JCM10317 | JN942668 | 676/774 (87.3%) |
| *Ustilago bouriqueti* | MS315 | AY740167 | 662/743 (89.1%) |
| *Ustilago vetiveriae* | H.U.V.17954 | AY345011 | 632/712 (88.8%) |
| *Sporisorium trachypogonis-plumosi* | MS281 | AY740060 | 634/700 (90.6%) |
| *Ustilago esculenta* | MS83 | AY345002 | 548/577 (95.0%) |
| *Ustilago hordei* | Ust.exs.784 | AY345003 | 660/763 (86.5%) |

### [Table 25]

**Table 25**

| Registration name | Strain name | Accession No. | Homology rate |
|---|---|---|---|
| *Dirkmeia sp.* | Y75 | MN515015 | 732/732 (100.0%) |
| *Dirkmeia churashimaensis* | Y78 | MN515013 | 732/732 (100.0%) |
| uncultured fungus | - | KX515770 | 732/732 (100.0%) |
| uncultured fungus | - | KX515470 | 732/732 (100.0%) |
| uncultured fungus | - | KX515432 | 732/732 (100.0%) |
| *fungal sp.* | N40 | MW131977 | 732/732 (100.0%) |
| *Dirkmeia sp.* | Y6 | MN515021 | 731/732 (99.9%) |
| *Dirkmeia sp.* | Y70 | MN515018 | 731/732 (99.9%) |
| *Dirkmeia sp.* | Y79 | MN515012 | 731/732 (99.9%) |
| *Dirkmeia churashimaensis ** | UZ271 17 | MF062257 | 731/732 (99.9%) |
| *Dirkmeia sp.* | M19 | MK651592 | 723/723 (100.0%) |
| uncultured fungus | - | KX515635 | 728/731 (99.6%) |
| *Dirkmeia sp.* | - | MK294224 | 728/732 (99.5%) |
| *fungal sp.* | SV657 | KP757563 | 718/719 (99.9%) |
| *Dirkmeia sp.* | Y9 | MN515020 | 727/736 (98.8%) |
| uncultured fungus | - | KX515629 | 725/736 (98.5%) |
| uncultured fungus | - | KX515626 | 724/736 (98.4%) |
| uncultured fungus | - | KX515578 | 724/736 (98.4%) |
| uncultured fungus | - | KX515487 | 724/736 (98.4%) |
| *Dirkmeia churashimaensis ** | RGJ1 | KU564518 | 688/688 (100.0%) |
| *Dirkmeia churashimaensis ** | SQUCC SS1 | MK583597 | 682/684 (99.7%) |
| *Dirkmeia churashimaensis* | - | MN758679 | 678/680 (99.7%) |
| *Dirkmeia churashimaensis* | - | MN758678 | 678/680 (99.7%) |
| *Dirkmeia churashimaensis* | - | MN758677 | 678/680 (99.7%) |
| *Dirkmeia churashimaensis* | - | MN758676 | 678/680 (99.7%) |
| *Dirkmeia churashimaensis* | - | MN758675 | 678/680 (99.7%) |
| *Dirkmeia churashimaensis* | - | MN758674 | 678/680 (99.7%) |
| *Dirkmeia churashimaensis* | - | MN758673 | 678/680 (99.7%) |
| *Dirkmeia churashimaensis* | - | MN758672 | 678/680 (99.7%) |
| *Dirkmeia churashimaensis* | - | MN758671 | 678/680 (99.7%) |

Based on the above, EC592 strain was identified as Dirkmeia churashimaensis in the results of nucleotide sequence analysis for the 26S rDNA-D1/D2 region and the rDNA-ITS region.

The morphological properties of EC592 strain were examined through observation of the nature and morphology of the colonies. As a result of culturing on a YM plate medium for 4 days, yellowish orange to cream-colored colony nature with a smooth, butter-like, wet, and viscous surface was exhibited. It was confirmed that the nutritive cells were oval to ovoid in shape, and that the proliferation was through budding from the short stalk of the cell pole part. No formation of sexual reproductive organs was observed in the plate after about 3 weeks from the start of culturing.

Based on the molecular phylogenetic position and morphological properties as well as the measurement of ergothioneine production, EC592 strain was determined to be a novel microorganism attributed to Dirkmeia churashimaensis.

### (Molecular phylogenetic position and morphological properties of EB761 strain)

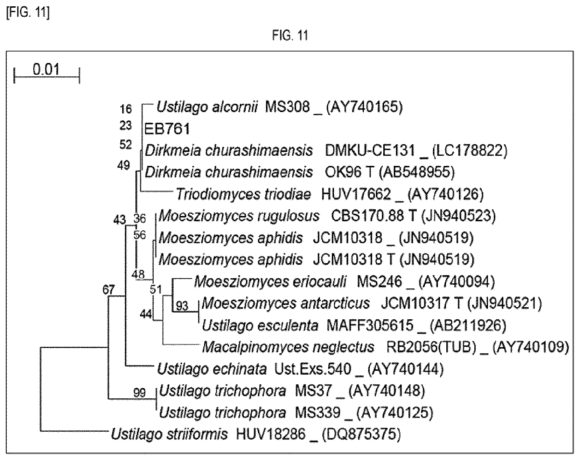

As a result of homology search by BLAST on DB-FU and International Nucleotide Sequence Databases using the microbial identification system "ENKI", the nucleotide sequence (SEQ ID NO: 11) of the 26S rDNA-D1/D2 region of EB761 strain showed 99.7 to 100% identity to a plurality of nucleotide sequences of Dirkmeia churashimaensis, which is a type of basidiomycete yeasts (Tables 26 and 27). In the molecular phylogenetic tree (FIG. 11) analyzed based on the nucleotide sequence obtained by homology search on DB-FU and International Nucleotide Sequence Databases, EB761 strain showed the same molecular phylogenetic position as that of a plurality of nucleotide sequences of Dirkmeia churashimaensis.

Table 26 shows the results of a search by BLAST on DB-FU, that is, the nucleotide sequence analysis data for the 26S rDNA-D1/D2 region found in the top 30 homology scores. The data with * is sequence data subjected to simplified molecular phylogenetic analysis.

Table 27 shows the results of a search by BLAST on International Nucleotide Sequence Databases, that is, the nucleotide sequence analysis data for the 26S rDNA-D1/D2 region found in the top 30 homology scores. The data with * is sequence data subjected to simplified molecular phylogenetic analysis. The strains with "^{a}" in Table 27 were excluded from the analysis because they are not with a nucleotide sequence derived from the reference strain, and the possibility of an error in the registration information was suggested.

FIG. 11 shows a simplified molecular phylogenetic tree based on the nucleotide sequence of the 26S rDNA-D1/D2 region of EB761 strain. The upper left line indicates the scale bar. The numbers positioned at the branches of the phylogenetic branches indicate bootstrap values. "T" at the end of a strain name indicates the type strain of the species.

### [Table 26]

**Table 26**

| Registration name | Strain name | Accession No. | Homology rate |
|---|---|---|---|
| *Dirkmeia churashimaensis ** | DMKU-CE131 | LC178822 | 605/605 (100.0%) |
| *Ustilago alcornii ** | MS308 | AY740165 | 603/605 (99.7%) |
| *Moesziomyces rupulosus ** | CBS170.88 | JN940523 | 603/605 (99.7%) |
| *Moesziomyces aphidis ** | JCM10318 | JN940519 | 603/605 (99.7%) |
| *Moesziomyces aphidis ** | JCM10318 | JN940519 | 603/605 (99.7%) |
| *Triodiomyces triodiae ** | HUV17662 | AY740126 | 602/605 (99.5%) |
| *Ustilago echinata ** | Ust.Exs.540 | AY740144 | 601/605 (99.3%) |
| *Moesziomyces eriocauli ** | MS246 | AY740094 | 599/605 (99.0%) |
| *Moesziomyces antarcticus ** | JCM10317 | JN940521 | 599/605 (99.0%) |
| *Macalpinomyces neglectus ** | RB2056(TUB) | AY740109 | 599/605 (99.0%) |
| *Ustilago esculenta ** | MAFF305615 | AB211926 | 598/604 (99.0%) |
| *Ustilago trichophora ** | MS37 | AY740148 | 598/605 (98.8%) |
| *Ustilago trichophora ** | MS339 | AY740125 | 598/605 (98.8%) |
| *Ustilago esculenta* | MS83 | AF453937 | 598/605 (98.8%) |
| *Ustilago affinis* | MP692 | AF133581 | 598/605 (98.8%) |
| *Moesziomyces bullatus* | CBS425.34 | DQ831011 | 598/605 (98.8%) |
| *Triodiomyces crassus* | CBS11336 | KY109978 | 599/605 (99.0%) |
| *Ustilago trichophora* | MP1898 | AJ236141 | 597/605 (98.7%) |
| *Ustilago calamagrostidis* | 56518[M] | AY740119 | 597/605 (98.7%) |
| *Sporisorium sorghi* | MP2036a | AF009872 | 599/607 (98.7%) |
| *Sporisorium cruentum* | MS14 | AY740156 | 599/607 (98.7%) |
| *Sporisorium andropoponis* | MS283 | AY740095 | 597/605 (98.7%) |
| *Anthracocystis elionuri* | MP2601 | AY740157 | 597/605 (98.7%) |
| *Anthracocystis provincialis* | Ust.exs.759(M) | AY747076 | 598/606 (98.7%) |
| *Ustilago crameri* | MS72 | AY740143 | 592/598 (99.0%) |
| *Sporisorium moniliferum* | MS98 | AF453940 | 597/605 (98.7%) |
| *Sporisorium foveolati* | MS21 | AY740103 | 598/607 (98.5%) |
| *Ustilago striiformis ** | HUV18286 | DQ875375 | 596/605 (98.5%) |
| *Sporisorium veracruzianum* | MP960 | AY740114 | 595/604 (98.5%) |
| *Sporisorium veracruzianum* | MP735 | AY740142 | 595/604 (98.5%) |

### [Table 27]

**Table 27**

| Registration name | Strain name | Accession No. | Homology rate |
|---|---|---|---|
| *Dirkmeia churashimaensis* | YE-162 | LC498467 | 605/605 (100.0%) |
| *Dirkmeia churashimaensis* | YE-157 | LC498464 | 605/605 (100.0%) |
| *Dirkmeia churashimaensis* | YE-149 | LC498460 | 605/605 (100.0%) |
| *Dirkmeia churashimaensis* | YE-121 | LC498442 | 605/605 (100.0%) |
| *Dirkmeia churashimaensis* | YE-36 | LC498414 | 605/605 (100.0%) |
| *Dirkmeia churashimaensis* | DMKU-RP81 | LC498270 | 605/605 (100.0%) |
| *Moesziomyces aphidis* ^{a} | UZ271 17 | MF062260 | 605/605 (100.0%) |
| *Dirkmeia churashimaensis* | DMKU-CE131 | LC178822 | 605/605 (100.0%) |
| *Dirkmeia churashimaensis* | DMKU-CE111 | LC178802 | 605/605 (100.0%) |
| *Dirkmeia churashimaensis* | DMKU-CE108 | LC178799 | 605/605 (100.0%) |
| *Dirkmeia churashimaensis* | DMKU-CE105 | LC178797 | 605/605 (100.0%) |
| *Dirkmeia churashimaensis* | DMKU-CE97 | LC178789 | 605/605 (100.0%) |
| *Dirkmeia churashimaensis* | DMKU-CE92 | LC178784 | 605/605 (100.0%) |
| *Dirkmeia churashimaensis* | DMKU-CE89 | LC178781 | 605/605 (100.0%) |
| *Dirkmeia churashimaensis* | DMKU-CE67 | LC178759 | 605/605 (100.0%) |
| *Dirkmeia churashimaensis* | DMKU-CE60 | LC178752 | 605/605 (100.0%) |
| *Dirkmeia churashimaensis* | DMKU-CE44 | LC178736 | 605/605 (100.0%) |
| *Dirkmeia churashimaensis* | DMKU-CE40 | LC178732 | 605/605 (100.0%) |
| *Dirkmeia churashimaensis* | DMKU-CE28 | LC178721 | 605/605 (100.0%) |
| *Dirkmeia churashimaensis* | DMKU-CE26 | LC178719 | 605/605 (100.0%) |
| *Dirkmeia churashimaensis* | DMKU-CE24 | LC178717 | 605/605 (100.0%) |
| *Dirkmeia churashimaensis* | DMKU-CE138 | LC178829 | 605/605 (100.0%) |
| *Pseudozyma sp.* | HB94-2 | KJ507299 | 605/605 (100.0%) |
| *Moesziomyces aphidis* ^{a} | LN05 | AB617892 | 605/605 (100.0%) |
| *Dirkmeia churashimaensis* | DMKU-CP709 | LC430833 | 604/604 (100.0%) |
| *Moesziomyces aphidis* | CBS 517.83 | NG069796 | 603/605 (99.7%) |
| *Dirkmeia churashimaensis* | 2Y95 | MT256146 | 603/605 (99.7%) |
| *Pseudozyma sp.* | - | MN533911 | 603/605 (99.7%) |
| *Dirkmeia churashimaensis* | YE-39 | LC498417 | 603/605 (99.7%) |
| *Moesziomyces aphidis* | CBS 517.83 | MH873352 | 603/605 (99.7%) |

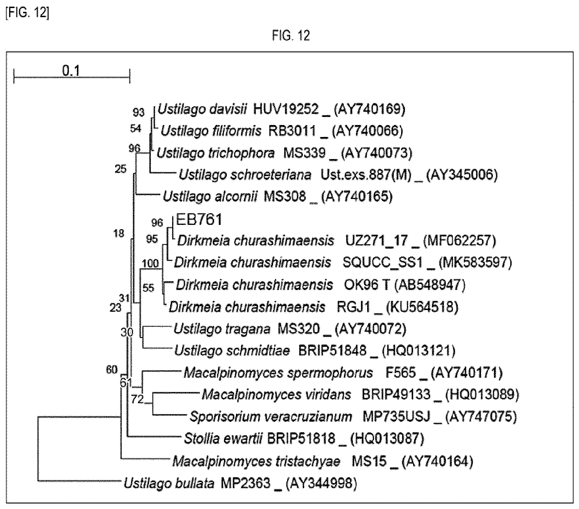

As a result of homology search by BLAST on DB-FU and International Nucleotide Sequence Databases using the microbial identification system "ENKI", the nucleotide sequence (SEQ ID NO: 12) of the rDNA-ITS region of EB761 strain showed 98.3 to 100% identity to a plurality of nucleotide sequences of Dirkmeia churashimaensis, which is a type of basidiomycete yeasts (Tables 28 and 29). In the molecular phylogenetic tree (FIG. 12) analyzed based on the nucleotide sequence obtained by homology search on DB-FU and International Nucleotide Sequence Databases, EB761 strain formed a cluster supported with a plurality of nucleotide sequences of Dirkmeia churashimaensis with a high bootstrap value of 100%.

Table 28 shows the results of a search by BLAST on DB-FU, that is, the nucleotide sequence analysis data for the rDNA-ITS region found in the top 30 homology scores. The data with * is sequence data subjected to simplified molecular phylogenetic analysis.

Table 29 shows the results of a search by BLAST on International Nucleotide Sequence Databases, that is, the nucleotide sequence analysis data for the rDNA-ITS region found in the top 30 homology scores. The data with * is sequence data subjected to simplified molecular phylogenetic analysis.

FIG. 12 shows a simplified molecular phylogenetic tree based on the nucleotide sequence of the rDNA-ITS region of EB761 strain. The upper left line indicates the scale bar. The numbers positioned at the branches of the phylogenetic branches indicate bootstrap values. "T" at the end of a strain name indicates the type strain of the species.

### [Table 28]

**Table 28**

| Registration name | Strain name | Accession No. | Homology rate |
|---|---|---|---|
| *Dirkmeia churashimaensis ** | OK96 | AB548947 | 691/703 (98.3%) |
| *Ustilago tragana ** | MS320 | AY740072 | 678/716 (94.7%) |
| *Ustilago davisii ** | HUV19252 | AY740169 | 683/728 (93.8%) |
| *Ustilago schmidtiae* * | BRIP51848 | HQ013121 | 688/749 (91.9%) |
| *Ustilago trichophora* * | MS339 | AY740073 | 672/717 (93.7%) |
| *Ustilago filiformis* * | RB3011 | AY740066 | 679/729 (93.1%) |
| *Ustilago alcornii ** | MS308 | AY740165 | 685/750 (91.3%) |
| *Macalpinomyces spermophorus* * | F565 | AY740171 | 667/734 (90.9%) |
| *Ustilago schroeteriana** | Ust.exs.887(M) | AY345006 | 672/751 (89.5%) |
| *Stollia ewartii ** | BRIP51818 | HQ013087 | 673/748 (90.0%) |
| *Macalpinomyces tristachyae ** | MS15 | AY740164 | 659/728 (90.5%) |
| *Macalpinomyces viridans ** | BRIP49133 | HQ013089 | 662/740 (89.5%) |
| *Sporisorium veracruzianum** | MP735USJ | AY747075 | 638/699 (91.3%) |
| *Macalpinomyces loudetiae* | MS250 | AY740151 | 649/721 (90.0%) |
| *Ustilago striiformis* | H.U.V.18286 | AY740172 | 664/754 (88.1%) |
| *Ustilago bullata ** | MP2363 | AY344998 | 673/768 (87.6%) |
| *Pseudozyma hubeiensis* | CBS10077 | DQ008954 | 636/705 (90.2%) |
| *Pseudozyma alboarmeniaca* | DMST17135 | AB117961 | 643/708 (90.8%) |
| *Stollia bursa* | KVU844 | JN367291 | 614/668 (91.9%) |
| *Ustilago pamirica* | Ust.exs.789(M) | AY345005 | 660/751 (87.9%) |
| *Ustilago austro-africana* | MS316 | AY740061 | 657/747 (88.0%) |
| *Macalpinomyces trichopterygis* | MS248 | AY740039 | 629/703 (89.5%) |
| *Sporisorium veracruzianum* | MP960 | AY344993 | 611/669 (91.3%) |
| *Ustilago vetiveriae* | H.U.V.17954 | AY345011 | 632/712 (88.8%) |
| *Ustilago bouriqueti* | MS315 | AY740167 | 661/743 (89.0%) |
| *Ustilago esculenta* | MAFF305615 | AB211926 | 675/774 (87.2%) |
| *Moesziomyces antarcticus* | JCM10317 | JN942668 | 675/774 (87.2%) |
| *Ustilago esculenta* | MS83 | AY345002 | 548/577 (95.0%) |
| *Sporisorium trachypogonis-plumosi* | MS281 | AY740060 | 634/700 (90.6%) |
| *Ustilago hordei* | Ust.exs.784 | AY345003 | 659/763 (86.4%) |

### [Table 29]

**Table 29**

| Registration name | Strain name | Accession No. | Homology rate |
|---|---|---|---|
| *Dirkmeia sp.* | Y75 | MN515015 | 731/732 (99.9%) |
| *Dirkmeia churashimaensis* | Y78 | MN515013 | 731/732 (99.9%) |
| uncultured fungus | - | KX515770 | 731/732 (99.9%) |
| uncultured fungus | - | KX515470 | 731/732 (99.9%) |
| uncultured fungus | - | KX515432 | 731/732 (99.9%) |
| *Dirkmeia sp.* | Y6 | MN515021 | 730/732 (99.7%) |
| *Dirkmeia sp.* | Y70 | MN515018 | 730/732 (99.7%) |
| *Dirkmeia sp.* | Y79 | MN515012 | 730/732 (99.7%) |
| *Dirkmeia churashimaensis ** | UZ271 17 | MF062257 | 730/732 (99.7%) |
| *Dirkmeia sp.* | M19 | MK651592 | 722/723 (99.9%) |
| uncultured fungus | - | KX515635 | 727/731 (99.5%) |
| *Dirkmeia sp.* | - | MK294224 | 727/732 (99.3%) |
| *fungal sp.* | SV657 | KP757563 | 717/719 (99.7%) |
| *Dirkmeia sp.* | Y9 | MN515020 | 726/736 (98.6%) |
| uncultured fungus | - | KX515629 | 724/736 (98.4%) |
| uncultured fungus | - | KX515626 | 723/736 (98.2%) |
| uncultured fungus | - | KX515578 | 723/736 (98.2%) |
| uncultured fungus | - | KX515487 | 723/736 (98.2%) |
| *Dirkmeia churashimaensis ** | RGJ1 | KU564518 | 688/688 (100.0%) |
| *Dirkmeia churashimaensis ** | SQUCC SS1 | MK583597 | 681/684 (99.6%) |
| *Dirkmeia churashimaensis* | - | MN758679 | 677/680 (99.6%) |
| *Dirkmeia churashimaensis* | - | MN758678 | 677/680 (99.6%) |
| *Dirkmeia churashimaensis* | - | MN758677 | 677/680 (99.6%) |
| *Dirkmeia churashimaensis* | - | MN758676 | 677/680 (99.6%) |
| *Dirkmeia churashimaensis* | - | MN758675 | 677/680 (99.6%) |
| *Dirkmeia churashimaensis* | - | MN758674 | 677/680 (99.6%) |
| *Dirkmeia churashimaensis* | - | MN758673 | 677/680 (99.6%) |
| *Dirkmeia churashimaensis* | - | MN758672 | 677/680 (99.6%) |
| *Dirkmeia churashimaensis* | - | MN758671 | 677/680 (99.6%) |
| *Dirkmeia churashimaensis* | - | MN758670 | 677/680 (99.6%) |

Based on the above, EB761 strain was identified as Dirkmeia churashimaensis in the results of nucleotide sequence analysis for the 26S rDNA-D1/D2 region and the rDNA-ITS region.

The morphological properties of EB761 strain were examined through observation of the nature and morphology of the colonies. As a result of culturing on a YM plate medium for 2 days, yellowish orange to cream-colored colony nature with a smooth, butter-like, and wet surface was exhibited. It was confirmed that the nutritive cells on Day 3 were oval to ovoid in shape, and that the proliferation was through budding from the short stalk of the cell pole part. No formation of sexual reproductive organs was observed in the plate after about 5 weeks from the start of culturing.

Based on the molecular phylogenetic position and morphological properties as well as the measurement of ergothioneine production, EB761 strain was determined to be a novel microorganism attributed to Dirkmeia churashimaensis.

### (Molecular phylogenetic position and morphological properties of EC021 strain)

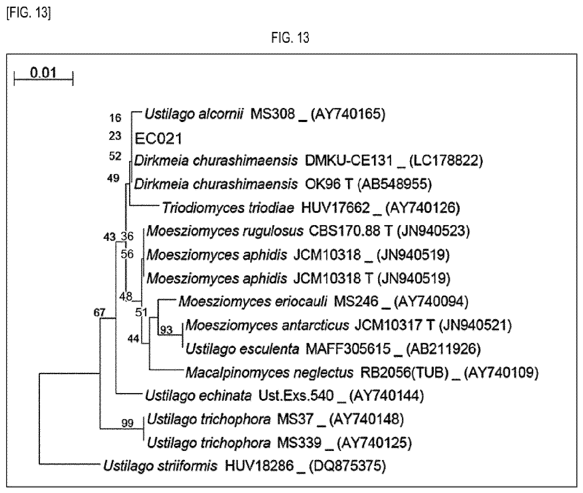

As a result of homology search by BLAST on DB-FU and International Nucleotide Sequence Databases using the microbial identification system "ENKI", the nucleotide sequence (SEQ ID NO: 13) of the 26S rDNA-D1/D2 region of EC021 strain showed 99.7 to 100% identity to a plurality of nucleotide sequences of Dirkmeia churashimaensis, which is a type of basidiomycete yeasts (Tables 30 and 31). In the molecular phylogenetic tree (FIG. 13) analyzed based on the nucleotide sequence obtained by homology search on DB-FU and International Nucleotide Sequence Databases, EC021 strain showed the same molecular phylogenetic position as that of a plurality of nucleotide sequences of Dirkmeia churashimaensis.

Table 30 shows the results of a search by BLAST on DB-FU, that is, the nucleotide sequence analysis data for the 26S rDNA-D1/D2 region found in the top 30 homology scores. The data with * is sequence data subjected to simplified molecular phylogenetic analysis.

Table 31 shows the results of a search by BLAST on International Nucleotide Sequence Databases, that is, the nucleotide sequence analysis data for the 26S rDNA-D1/D2 region found in the top 30 homology scores. The data with * is sequence data subjected to simplified molecular phylogenetic analysis. The strains with "^{a}" in Table 31 were excluded from the analysis because they are not with a nucleotide sequence derived from the reference strain, and the possibility of an error in the registration information was suggested.

FIG. 13 shows a simplified molecular phylogenetic tree based on the nucleotide sequence of the 26S rDNA-D1/D2 region of EC021 strain. The upper left line indicates the scale bar. The numbers positioned at the branches of the phylogenetic branches indicate bootstrap values. "T" at the end of a strain name indicates the type strain of the species.

### [Table 30]

**Table 30**

| Registration name | Strain name | Accession No. | Homology rate |
|---|---|---|---|
| *Dirkmeia churashimaensis ** | DMKU-CE131 | LC178822 | 605/605 (100.0%) |
| *Ustilago alcornii ** | MS308 | AY740165 | 603/605 (99.7%) |
| *Moesziomyces rugulosus ** | CBS170.88 | JN940523 | 603/605 (99.7%) |
| *Moesziomyces aphidis ** | JCM10318 | JN940519 | 603/605 (99.7%) |
| *Moesziomyces aphidis ** | JCM10318 | JN940519 | 603/605 (99.7%) |
| *Triodiomyces triodiae ** | HUV17662 | AY740126 | 602/605 (99.5%) |
| *Ustilago echinate ** | Ust.Exs.540 | AY740144 | 601/605 (99.3%) |
| *Moesziomyces eriocauli ** | MS246 | AY740094 | 599/605 (99.0%) |
| *Moesziomyces antarcticus ** | JCM10317 | JN940521 | 599/605 (99.0%) |
| *Macalpinomyces neglectus ** | RB2056(TUB) | AY740109 | 599/605 (99.0%) |
| *Ustilago esculenta ** | MAFF305615 | AB211926 | 598/604 (99.0%) |
| *Ustilago trichophora ** | MS37 | AY740148 | 598/605 (98.8%) |
| *Ustilago trichophora* * | MS339 | AY740125 | 598/605 (98.8%) |
| *Ustilago esculenta* | MS83 | AF453937 | 598/605 (98.8%) |
| *Ustilago affinis* | MP692 | AF133581 | 598/605 (98.8%) |
| *Moesziomyces bullatus* | CBS425.34 | DQ831011 | 598/605 (98.8%) |
| *Triodiomyces crassus* | CBS11336 | KY109978 | 599/605 (99.0%) |
| *Sporisorium sorghi* | MP2036a | AF009872 | 599/607 (98.7%) |
| *Sporisorium cruentum* | MS14 | AY740156 | 599/607 (98.7%) |
| *Ustilago trichophora* | MP1898 | AJ236141 | 597/605 (98.7%) |
| *Ustilago calamagrostidis* | 56518[M] | AY740119 | 597/605 (98.7%) |
| *Sporisorium andropogonis* | MS283 | AY740095 | 597/605 (98.7%) |
| *Anthracocystis elionuri* | MP2601 | AY740157 | 597/605 (98.7%) |
| *Anthracocystis provincialis* | Ust.exs.759(M) | AY747076 | 598/606 (98.7%) |
| *Sporisorium moniliferum* | MS98 | AF453940 | 597/605 (98.7%) |
| *Ustilago crameri* | MS72 | AY740143 | 592/598 (99.0%) |
| *Sporisorium foveolati* | MS21 | AY740103 | 598/607 (98.5%) |
| *Ustilago striiformis ** | HUV18286 | DQ875375 | 596/605 (98.5%) |
| *Sporisorium veracruzianum* | MP960 | AY740114 | 595/604 (98.5%) |
| *Sporisorium veracruzianum* | MP735 | AY740142 | 595/604 (98.5%) |

### [Table 31]

**Table 31**

| Registration name | Strain name | Accession No. | Homology rate |
|---|---|---|---|
| *Dirkmeia churashimaensis* | YE-162 | LC498467 | 605/605 (100.0%) |
| *Dirkmeia churashimaensis* | YE-157 | LC498464 | 605/605 (100.0%) |
| *Dirkmeia churashimaensis* | YE-149 | LC498460 | 605/605 (100.0%) |
| *Dirkmeia churashimaensis* | YE-121 | LC498442 | 605/605 (100.0%) |
| *Dirkmeia churashimaensis* | YE-36 | LC498414 | 605/605 (100.0%) |
| *Dirkmeia churashimaensis* | DMKU-RP81 | LC498270 | 605/605 (100.0%) |
| *Moesziomyces aphidis* ^{a} | - | MF062260 | 605/605 (100.0%) |
| *Dirkmeia churashimaensis* | DMKU-CE131 | LC178822 | 605/605 (100.0%) |
| *Dirkmeia churashimaensis* | DMKU-CE111 | LC178802 | 605/605 (100.0%) |
| *Dirkmeia churashimaensis* | DMKU-CE108 | LC178799 | 605/605 (100.0%) |
| *Dirkmeia churashimaensis* | DMKU-CE105 | LC178797 | 605/605 (100.0%) |
| *Dirkmeia churashimaensis* | DMKU-CE97 | LC178789 | 605/605 (100.0%) |
| *Dirkmeia churashimaensis* | DMKU-CE92 | LC178784 | 605/605 (100.0%) |
| *Dirkmeia churashimaensis* | DMKU-CE89 | LC178781 | 605/605 (100.0%) |
| *Dirkmeia churashimaensis* | DMKU-CE67 | LC178759 | 605/605 (100.0%) |
| *Dirkmeia churashimaensis* | DMKU-CE60 | LC178752 | 605/605 (100.0%) |
| *Dirkmeia churashimaensis* | - | LC178736 | 605/605 (100.0%) |
| *Dirkmeia churashimaensis* | DMKU-CE40 | LC178732 | 605/605 (100.0%) |
| *Dirkmeia churashimaensis* | DMKU-CE28 | LC178721 | 605/605 (100.0%) |
| *Dirkmeia churashimaensis* | DMKU-CE26 | LC178719 | 605/605 (100.0%) |
| *Dirkmeia churashimaensis* | DMKU-CE24 | LC178717 | 605/605 (100.0%) |
| *Dirkmeia churashimaensis* | DMKU-CE138 | LC178829 | 605/605 (100.0%) |
| *Pseudozyma sp.* | HB94-2 | KJ507299 | 605/605 (100.0%) |
| *fungal sp.* | N40 | MW131977 | 605/605 (100.0%) |
| *Moesziomyces aphidis* ^{a} | LN05 | AB617892 | 605/605 (100.0%) |
| *Dirkmeia churashimaensis* | DMKU-CP709 | LC430833 | 604/604 (100.0%) |
| *Moesziomyces aphidis* | DMKU-RK86 | AB772475 | 603/605 (99.7%) |
| *Moesziomyces aphidis* | CBS 517.83 | NG069796 | 603/605 (99.7%) |
| *Dirkmeia churashimaensis* | - | MT256146 | 603/605 (99.7%) |
| *Pseudozyma sp.* | - | MN533911 | 603/605 (99.7%) |

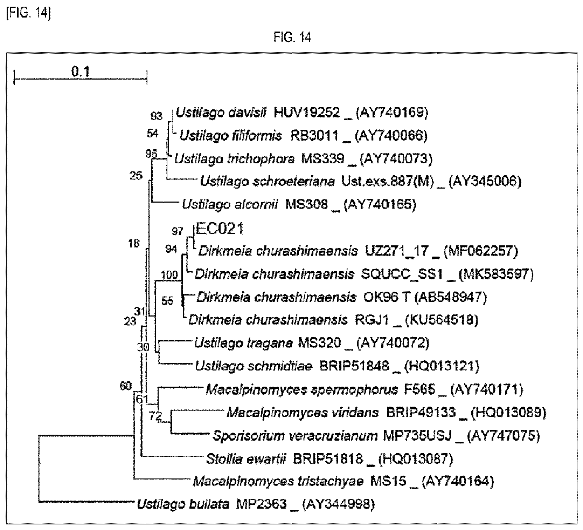

As a result of homology search by BLAST on DB-FU and International Nucleotide Sequence Databases using the microbial identification system "ENKI", the nucleotide sequence (SEQ ID NO: 14) of the rDNA-ITS region of EC021 strain showed 98.4 to 100% identity to a plurality of nucleotide sequences of Dirkmeia churashimaensis, which is a type of basidiomycete yeasts (Tables 32 and 33). In the molecular phylogenetic tree (FIG. 14) analyzed based on the nucleotide sequence obtained by homology search on DB-FU and International Nucleotide Sequence Databases, EC021 strain formed a cluster supported with a plurality of nucleotide sequences of Dirkmeia churashimaensis with a high bootstrap value of 100%.

Table 32 shows the results of a search by BLAST on DB-FU, that is, the nucleotide sequence analysis data for the rDNA-ITS region found in the top 30 homology scores. The data with * is sequence data subjected to simplified molecular phylogenetic analysis.

Table 33 shows the results of a search by BLAST on International Nucleotide Sequence Databases, that is, the nucleotide sequence analysis data for the rDNA-ITS region found in the top 30 homology scores. The data with * is sequence data subjected to simplified molecular phylogenetic analysis.

FIG. 14 shows a simplified molecular phylogenetic tree based on the nucleotide sequence of the rDNA-ITS region of EC021 strain. The upper left line indicates the scale bar. The numbers positioned at the branches of the phylogenetic branches indicate bootstrap values. "T" at the end of a strain name indicates the type strain of the species.

### [Table 32]

**Table 32**

| Registration name | Strain name | Accession No. | Homology rate |
|---|---|---|---|
| *Dirkmeia churashimaensis ** | OK96 | AB548947 | 692/703 (98.4%) |
| *Ustilago tragana ** | MS320 | AY740072 | 679/716 (94.8%) |
| *Ustilago davisii ** | HUV19252 | AY740169 | 683/728 (93.8%) |
| *Ustilago schmidtiae* * | BRIP51848 | HQ013121 | 689/749 (92.0%) |
| *Ustilago trichophora* * | MS339 | AY740073 | 673/717 (93.9%) |
| *Ustilago filiformis* * | RB3011 | AY740066 | 680/729 (93.3%) |
| *Ustilago alcornii ** | MS308 | AY740165 | 686/750 (91.5%) |
| *Macalpinomyces spermophorus* * | F565 | AY740171 | 668/734 (91.0%) |
| *Stollia ewartii ** | BRIP51818 | HQ013087 | 675/748 (90.2%) |
| *Macalpinomyces tristachyae ** | MS15 | AY740164 | 661/728 (90.8%) |
| *Ustilago schroeteriana** | Ust.exs.887(M) | AY345006 | 672/751 (89.5%) |
| *Macalpinomyces viridans* * | BRIP49133 | HQ013089 | 663/740 (89.6%) |
| *Sporisorium veracruzianum** | MP735USJ | AY747075 | 639/699 (91.4%) |
| *Macalpinomyces loudetiae* | MS250 | AY740151 | 650/721 (90.2%) |
| *Ustilago bullata ** | MP2363 | AY344998 | 674/768 (87.8%) |
| *Ustilago striiformis* | H.U.V.18286 | AY740172 | 667/754 (88.5%) |
| *Pseudozyma hubeiensis* | CBS10077 | DQ008954 | 637/705 (90.4%) |
| *Pseudozyma alboarmeniaca* | DMST17135 | AB117961 | 644/708 (91.0%) |
| *Ustilago austro-africana* | MS316 | AY740061 | 659/747 (88.2%) |
| *Ustilago pamirica* | Ust.exs.789(M) | AY345005 | 661/751 (88.0%) |
| *Stollia bursa* | KVU844 | JN367291 | 614/668 (91.9%) |
| *Macalpinomyces trichopterygis* | MS248 | AY740039 | 629/703 (89.5%) |
| *Ustilago esculenta* | MAFF305615 | AB211926 | 676/774 (87.3%) |
| *Sporisorium veracruzianum* | MP960 | AY344993 | 611/669 (91.3%) |
| *Moesziomyces antarcticus* | JCM10317 | JN942668 | 676/774 (87.3%) |
| *Ustilago bouriqueti* | MS315 | AY740167 | 662/743 (89.1%) |
| *Ustilago vetiveriae* | H.U.V.17954 | AY345011 | 632/712 (88.8%) |
| *Sporisorium trachypogonis-plumosi* | MS281 | AY740060 | 634/700 (90.6%) |
| *Ustilago esculenta* | MS83 | AY345002 | 548/577 (95.0%) |
| *Ustilago hordei* | Ust.exs.784 | AY345003 | 660/763 (86.5%) |

### [Table 33]

**Table 33**

| Registration name | Strain name | Accession No. | Homology rate |
|---|---|---|---|
| *Dirkmeia sp.* | Y75 | MN515015 | 732/732 (100.0%) |
| *Dirkmeia churashimaensis* | Y78 | MN515013 | 732/732 (100.0%) |
| uncultured fungus | - | KX515770 | 732/732 (100.0%) |
| uncultured fungus | - | KX515470 | 732/732 (100.0%) |
| uncultured fungus | - | KX515432 | 732/732 (100.0%) |
| *fungal sp.* | N40 | MW131977 | 732/732 (100.0%) |
| *Dirkmeia sp.* | Y6 | MN515021 | 731/732 (99.9%) |
| *Dirkmeia sp.* | Y70 | MN515018 | 731/732 (99.9%) |
| *Dirkmeia sp.* | - | MN515012 | 731/732 (99.9%) |
| *Dirkmeia churashimaensis ** | UZ271 17 | MF062257 | 731/732 (99.9%) |
| *Dirkmeia sp.* | M19 | MK651592 | 723/723 (100.0%) |
| uncultured fungus | - | KX515635 | 728/731 (99.6%) |
| *Dirkmeia sp.* | - | MK294224 | 728/732 (99.5%) |
| *fungal sp.* | SV657 | KP757563 | 718/719 (99.9%) |
| *Dirkmeia sp.* | Y9 | MN515020 | 727/736 (98.8%) |
| uncultured fungus | - | KX515629 | 725/736 (98.5%) |
| uncultured fungus | - | KX515626 | 724/736 (98.4%) |
| uncultured fungus | - | KX515578 | 724/736 (98.4%) |
| uncultured fungus | - | KX515487 | 724/736 (98.4%) |
| *Dirkmeia churashimaensis ** | RGJ1 | KU564518 | 688/688 (100.0%) |
| *Dirkmeia churashimaensis ** | SQUCC SS1 | MK583597 | 682/684 (99.7%) |
| *Dirkmeia churashimaensis* | - | MN758679 | 678/680 (99.7%) |
| *Dirkmeia churashimaensis* | - | MN758678 | 678/680 (99.7%) |
| *Dirkmeia churashimaensis* | - | MN758677 | 678/680 (99.7%) |
| *Dirkmeia churashimaensis* | - | MN758676 | 678/680 (99.7%) |
| *Dirkmeia churashimaensis* | - | MN758675 | 678/680 (99.7%) |
| *Dirkmeia churashimaensis* | - | MN758674 | 678/680 (99.7%) |
| *Dirkmeia churashimaensis* | - | MN758673 | 678/680 (99.7%) |
| *Dirkmeia churashimaensis* | - | MN758672 | 678/680 (99.7%) |
| *Dirkmeia churashimaensis* | - | MN758671 | 678/680 (99.7%) |

Based on the above, EC021 strain was identified as Dirkmeia churashimaensis in the results of nucleotide sequence analysis for the 26S rDNA-D1/D2 region and the rDNA-ITS region.

The morphological properties of EC021 strain were examined through observation of the nature and morphology of the colonies. As a result of culturing on a YM plate medium for 3 days, yellowish orange to cream-colored colony nature with a smooth, butter-like, wet, and viscous surface was exhibited. It was confirmed that the nutritive cells were oval to ovoid in shape, and that the proliferation was through budding from the short stalk from the cell local part. No formation of sexual reproductive organs was observed in the plate after about 3 weeks from the start of culturing.

Based on the molecular phylogenetic position and morphological properties as well as the measurement of ergothioneine production, EC021 strain was determined to be a novel microorganism attributed to Dirkmeia churashimaensis.

### INDUSTRIAL APPLICABILITY

The microorganisms of the present invention have high ergothioneine production and can be used in the fields of health, beauty, and the like.

### ACCESSION NUMBER

NITE BP-03706
NITE BP-03707
NITE BP-03708
NITE BP-03709
NITE BP-03710
NITE BP-03711
NITE BP-03712

## Claims

1. A microorganism belonging to Dirkmeia churashimaensis (accession number: NITE BP-03707, accession number: NITE BP-03708, accession number: NITE BP-03709, accession number: NITE BP-03711, or accession number: NITE BP-03712), a microorganism belonging to Aureobasidium melanogenum (accession number: NITE BP-03706), or a microorganism belonging to a species closely related to Ustilago sporoboli-indici (Ustilago sp.) (accession number: NITE BP-03710).

2. A culture of the microorganism described in claim 1.

3. An extract of the microorganism described in claim 1.

4. A method for producing ergothioneine, the method comprising culturing the microorganism described in claim 1 to obtain a culture containing ergothioneine.
